# EUROPÄISCHE PATENTSCHRIFT

(11) **EP 1 519 766 B1**
(45) Veröffentlichungstag und Bekanntmachung des Hinweises auf die Patenterteilung: **24.09.2008**
(21) Anmeldenummer: 03761386.6
(22) Anmeldetag: 25.06.2003
(51) Int. Cl.: A61M 5/315

(54) **PRODUKTAUSSCHÜTTVORRICHTUNG MIT KOLBENSTANGEN-EILRÜCKSETZUNG**
PRODUCT DISTRIBUTION DEVICE WITH RAPID PISTON ROD RESETTING
DISPOSITIF DE DEVERSEMENT DE PRODUIT COMPORTANT UN MECANISME DE RETOUR RAPIDE DE LA TIGE DE PISTON

(30) Priorität: 28.06.2002 DE 10229138
(43) Veröffentlichungstag der Anmeldung: 06.04.2005
(73) Patentinhaber: TecPharma Licensing AG, 3401 Burgdorf (CH)
(72) Erfinder: HOMMANN, Edgar, CH-3257 GROSSAFFOLTERN (CH)
(74) Vertreter: Schwabe - Sandmair - Marx
(86) Internationale Anmeldenummer: PCT/CH2003/000419
(87) Internationale Veröffentlichungsnummer: WO 2004/002556

(56) Entgegenhaltungen:
- EP-A- 0 295 075
- EP-A- 0 327 910
- EP-A- 0 450 905
- EP-A- 0 956 874
- WO-A-01/72361
- WO-A-02/30495
- WO-A-88/08724
- WO-A-91/10460
- WO-A-96/07443
- US-A- 5 514 097
- US-A- 5 591 136
- US-B1- 6 277 097

## Beschreibung

Die Erfindung betrifft eine Vorrichtung für eine dosierte Ausschüttung eines Produkts. Das Produkt kann unmittelbar bei der Ausschüttung verabreicht werden, beispielsweise durch Injektion. Die Vorrichtung kann aber auch in Verbindung mit einer Verabreichungseinrichtung verwendet werden, beispielsweise in Verbindung mit einer Druckinjektionseinrichtung oder insbesondere einer Zersteubungseinrichtung eines Inhalators. Mit der Produktausschüttung wird im zweiten Verwendungsfall die gewünschte Produktdosis eingestellt und in die nachgeschaltete, eigentliche Verabreichungseinrichtung ausgeschüttet.

Bekannte Injektionsgeräte, wie eines beispielsweise in der WO 99/03522 beschrieben wird, weisen in einem produktgefüllten Reservoir einen Kolben auf, um das Produkt aus dem Reservoir auszuschütten. Die Ausschüttbewegung des Kolbens wird mittels einer Kolbenstange bewirkt. Für die Auswahl der auszuschüttenden Produktdosis und die Ausschüttung selbst umfassen die bekannten Geräte ferner eine Dosier- und Betätigungseinrichtung, die mit der Kolbenstange gekoppelt ist. Die bekannten Dosier- und Betätigungseinrichtungen umfassen ein Dosierglied, das unmittelbar mit der Kolbenstange in einem Eingriff ist. Die Kolbenstange wird durch die Dosisauswahl relativ zu den Dosierglied axial ein Stück weit in Richtung auf den Kolben vorbewegt. Anschließend wird die Dosier- und Betätigungseinrichtung mit dem Dosierglied und der Kolbenstange vorgeschoben. Bei dieser Ausschüttbewegung drückt die Kolbenstange gegen den Kolben, der deshalb in dem Reservoir auf einen Reservoirauslass zu vorschiebt, wodurch die gegen den Kolben, der deshalb in dem Reservoir auf einen Reservoirauslass zu vorschiebt, wodurch die ausgewählte Produktdosis ausgeschüttet wird. Der Vorgang der Dosisauswahl und Produktausschüttung kann wiederholt werden bis die für die Dosisauswahl verfügbare Länge der Kolbenstange aufgebraucht ist. Bei der Vorrichtung der WO 99/03522 ist die Kolbenstange eine Gewindestange und das Dosierglied eine Gewindemutter, die miteinander in einen Gewindeeingriff sind. Soll die Kolbenstange nach einem Auffüllen oder einem Austausch des Reservoirs für die weitere Produktverabreichung erneut verwendet werden, so muss sie über ihre für die Dosisauswahl verbrauchte Gewindelänge relativ zu dem Dosierglied zurückgedreht werden, was umständlich ist.

Die WO 01/72361 A1 offenbart eine Injektionsvorrichtung, bei der für eine Ausschüttbewegung zwei kugelförmige Eingriffsglieder in eine Kolbenstange eingreifen. Die Ausschüttbewegung wird von einem Vorschubelement über die Kugeln auf die Kolbenstange übertragen. Ferner sind bei der Vorrichtung Backen vorgesehen, die ebenfalls in die Kolbenstange eingreifen und eine Bewegung der Kolbenstange in die der Ausschüttbewegung entgegengesetzte Richtung, wie z. B. bei der Vornahme einer Dosierung, verhindern. Um ein neues Produktbehältnis in die Vorrichtung einsetzen zu können, muss die Kolbenstange wieder zurück in proximale Richtung geschoben werden. Dabei werden die Backen aus dem Eingriff mit der Kolbenstange gelöst, so dass eine Eilrücksetzung der Kolbenstange möglich ist.

Bei einer anderen bekannten Bauart von Produktausschüttvorrichtungen ist die Kolbenstange als Zahnstange und das Dosierglied als elastischer Schnapper ausgebildet, der in die Sägezähne der Kolbenstange eingreift. Bei Vorrichtungen dieser Art ist eine Rücksetzung der Kolbenstange im Allgemeinen überhaupt nicht möglich.

Es ist eine Aufgabe der Erfindung, eine Produktausschüttvorrichtung zu schaffen, die nach Vornahme einer Dosisauswahl rasch und bequem wieder in einen Ausgangszustand zurückgesetzt werden kann, den sie vor der Dosisauswahl eingenommen hat.

Eine Produktausschüttvorrichtung, wie die Erfindung sie betrifft, weist ein Gehäuse, eine Kolbenstange, eine Dosier- und Betätigungseinrichtung und ein Dosierglied auf, das mit der Kolbenstange in einem Eingriff ist. Das Gehäuse weist einen Gehäuseabschnitt auf, der der Aufnahme eines produktgefüllten Reservoirs dient. Der betreffende Gehäuseabschnitt kann das Reservoir unmittelbar bilden. Bevorzugter bildet er jedoch einen Halter für ein einsetzbares Produktbehältnis. Das Reservoir weist einen Auslass auf und wird an einem von dem Auslass abgewandten Ende von einem Kolben verschlossen, der in eine Vorschubrichtung auf den Auslass zu verschiebbar ist, um das Produkt durch den Auslass zu verdrängen. Der Kolben kann fester Bestandteil der Vorrichtung sein. Der Kolben kann insbesondere jedoch Bestandteil eines Produktbehältnisses und zusammen mit dem Behältnis austauschbar sein, wie dies in der Selbstverabreichung üblich ist. So stellt die Selbstverabreichung im Rahmen einer Therapie, beispielsweise die Verabreichung von Insulin oder Wachstumshormonen, ein bevorzugtes Anwendungsgebiet der Erfindung dar.

Die Dosier- und Betätigungseinrichtung wird von dem Gehäuse so gelagert, dass sie relativ zu dem Gehäuse eine Ausschüttbewegung in die Vorschubrichtung und eine Dosierbewegung ausführen kann. Das Dosierglied ist mit der Dosier- und Betätigungseinrichtung so in einem formschlüssigen Eingriff, dass es die Ausschüttbewegung exakt mitmacht. Ferner ist es mit der Kolbenstange in einem Eingriff. Der Eingriff der Dosier- und Betätigungseinrichtung mit dem Dosierglied und der Eingriff des Dosierglieds mit der Kolbenstange sind so, dass das Dosierglied durch die Dosierbewegung der Dosier- und Betätigungseinrichtung in eine Dosierbewegung relativ zu dem Gehäuse und relativ zu der Kolbenstange versetzt wird. So können die Dosier- und Betätigungseinrichtung und die Kolbenstange beispielsweise in Bezug auf eine Längsachse der Kolbenstange verdrehgesichert und axial relativ zueinander verschiebbar miteinander verbunden sein, während das Dosierglied im Gehäuse axial geradgeführt wird und mit der Kolbenstange in einem Gewindeeingriff ist. Die Dosierbewegung des Dosierglieds kann in diesem Fall eine Translationsbewegung gegen die Vorschubrichtung sein.

Vorzugsweise sind die Dosier- und Betätigungseinrichtung und das Dosierglied jedoch verdrehgesichert und in Bezug auf die Axialrichtung verschiebegesichert miteinander verbunden. Die Dosier- und Betätigungseinrichtung und das Dosierglied führen somit nicht nur die Ausschüttbewegung, sondern auch die Dosierbewegung gemeinsam aus. Der Eingriff, in dem das Dosierglied und die Kolbenstange sich befinden, lässt, wie in dem alternativen Beispielfall auch, die Dosierbewegung des Dosierglieds relativ zu der Kolbenstange zu und bewirkt die Mitnahme der Kolbenstange bei der Ausschüttbewegung.

In der DE 199 45 397 ist ein Kraftschlüssiger Eingriff des Dosierglieds mit der Kolbenstange beschrieben. Die Erfindung weist allerdings ein formschlüssiger Eingriff auf. Das Dosierglied ist ein Gewindeglied, das mit der Kolbenstange in einem Gewindeeingriff ist. Obgleich einem Gewindeeingriff deutlich der Vorzug gegeben wird, können die Kolbenstange statt dessen auch als Zahnstange und das Dosierglied als elastischer Schnapper ausgebildet sein, wie dies grundsätzlich bekannt ist.

Das Dosierglied ist quer zu der Kolbenstange bewegbar, um den für die Dosisauswahl und die Ausschüttung bestehenden Eingriff mit der Kolbenstange lösen zu können. Wenn der Eingriff gelöst ist, kann die Kolbenstange gegen die Vorschubrichtung bis in eine hinterste Ausgangsposition zurück bewegt, d.h. zurückgesetzt, werden. In der hintersten Ausgangsposition steht wieder die gesamte, für die Dosierung wirksame Länge der Kolbenstange zur Verfügung.

Falls das Dosierglied ein Gewindeglied und die Kolbenstange dementsprechend eine Gewindestange ist, umgreift das Dosierglied in dem Gewindeeingriff die Kolbenstange über einen im Bogenmaß gemessenen Umfang von höchstens 180°, um die Querbewegung relativ zu der Kolbenstange zu ermöglichen. In einer bevorzugten Ausbildung als Gewindeglied ist das Dosierglied als axial geteilte Gewindemutter gebildet, vorzugsweise als geteilte Gewindemutter bestehend aus zwei Mutternhälften, die quer zu der Kolbenstange in entgegengesetzte Richtungen aus dem Eingriff bewegbar angeordnet sind.

Falls die Kolbenstange eine Zahnstange und das Dosierglied ein elastisch von der Kolbenstange abbiegbarer Schnapper oder eine Schnappereinrichtung mit mehreren solcher Schnapper ist, wird unter der Querbewegung des Dosierglieds zum Lösen des Eingriffs mit der Kolbenstange nicht die bei solchen Dosiergliedem übliche elastische Abbiegung zum Zwecke der Dosierung verstanden, sondern eine Querbewegung, bei der der oder die Schnapper bis in eine Ausrückposition bewegt werden, in der sie nicht durch ihre Elastizitätskraft in die Zahnlücken des Zahnstangenabschnitts der Kolbenstange hinein ragen, sondern frei von der Kolbenstange sind.

Die erfindungsgemäße Vorrichtung umfasst ferner ein Rücksetz-Betätigungselement, das mit dem Gehäuse derart verbunden ist, das es relativ zu dem Gehäuse eine Ausrück- und eine Einrückbewegung ausführen kann. Das Rücksetz-Betätigungselement bildet in bevorzugter Ausführung selbst einen zweiten Gehäuseabschnitt. Grundsätzlich kann es aber auch ein zusätzliches Element sein, das an dem Gehäuse bewegbar angebracht ist.

Das Rücksetz-Betätigungselement und das Dosierglied werden von einem Kurvengetriebe miteinander gekoppelt. Das Kurvengetriebe wandelt die Ausrückbewegung des Rücksetz-Betätigungselements in die Querbewegung des Dosierglieds um, die das Dosierglied aus dem mit der Kolbenstange bestehenden Eingriff führt. Ferner wandelt das Kurvengetriebe in die andere Richtung auch die Einrückbewegung des Rücksetz-Betätigungselements in die in den Eingriff mit der Kolbenstange zurückführende Querbewegung des Dosierglieds um. Die Querbewegung des Dosierglieds ist eine durch Formschluss geführte Bewegung entlang einer quer zu der Längsachse weisenden Führungsbahn, die im Folgenden als Querführungsbahn bezeichnet wird. Vorzugsweise wird unmittelbar das Dosierglied an der Querführungsbahn geführt. Das Dosierglied kann in dieser Ausführung einen Gleitstück bilden. Die Querführungsbahn schließt das Dosierglied oder gegebenenfalls ein mit dem Dosierglied verbundenes und an der Querführungsbahn geführtes Übertragungsstück vorzugsweise beidseitig ein, so dass Bewegungen des Dosierglieds in oder gegen die Vorschubrichtung bei der Querbewegung in und aus dem Eingriff mit der Kolbenstange bereits durch die Führung an der Querführungsbahn verhindert werden.

Mittels des Kurvengetriebes, das mit dem Rücksetz-Betätigungselement und mit dem Dosierglied gekoppelt ist, kann die Rücksetzung der Kolbenstange bei geschlossenem Gehäuse vorgenommen werden. Es muss lediglich das Rücksetz-Betätigungselement relativ zu dem Gehäuse so betätigt werden, dass es seine Ausrückbewegung ausführt. Die Ausrückbewegung wird von dem Kurvengetriebe automatisch in die den Eingriff mit der Kolbenstange lösende Querbewegung des Dosierglieds übertragen. Gegebenenfalls wird die Vorrichtung noch in eine Position gebracht, in der bei gelöstem Eingriff die Kolbenstange unter der Wirkung der Schwerkraft gegen die Vorschubrichtung in eine Ausgangsposition fällt oder vorzugsweise gleitet.

In bevorzugten Ausführungen wird das Rücksetz-Betätigungselement, wie bereits erwähnt, von einem zweiten Gehäuseabschnitt gebildet. Der erste und der zweite Gehäuseabschnitt können Hülsenteile sein, die miteinander verschraubt sind. Dabei handelt es sich vorzugsweise um die beiden Gehäuseabschnitte, die für einen Reservoiraustausch oder ein Nachfüllen des Reservoirs sowieso auseinander geschraubt werden müssen. Die bei dem Auseinanderschrauben ausgeführte Relativbewegung zwischen den beiden Gehäuseabschnitten kann nämlich gleichzeitig auch die Ausrückbewegung sein. Die zwei Gehäuseabschnitte können grundsätzlich auch mittels einer Steckverbindung und/oder Rastverbindung miteinander verbunden sein, die eine Drehbewegung der Gehäuseabschnitte relativ zueinander nicht erfordert. Eine Verbindung mittels Gewindeeingriff ist zwischen den Gehäuseabschnitte jedoch zu bevorzugen, da hierdurch die Ausrückbewegung und die Einrückbewegung besonders fein kontrolliert ausgeführt werden können.

Grundsätzlich muss das Rücksetz-Betätigungselement nicht selbst einen Gehäuseabschnitt bilden. Es kann auch ein Zusatzteil sein, das vorzugsweise mittels Gewindeeingriff mit dem Gehäuse, beispielsweise mit dem ersten Gehäuseabschnitt oder einem zweiten Gehäuseabschnitt, verbunden ist.

Vorzugsweise bildet das Kurvengetriebe für die Kopplung des Rücksetz-Betätigungselements mit dem Dosierglied wenigstens eine weitere Führungsbahn. Ein mit dem Rücksetz-Betätigungselement verbundenes Getriebeeingangsglied und ein mit dem Dosierglied verbundenes Getriebeausgangsglied bilden ein Kurvenglied und ein Eingriffsglied des Kurvengetriebes. Das Kurvenglied ist mit einer Führungsbahn versehen, an der das Eingriffsglied in einem Führungseingriff geführt wird. Durch diesen Führungseingriff werden die Ausrückbewegung und die Einrückbewegung des Rücksetz-Betätigungselements in die zugeordnete Querbewegung des Dosierglieds relativ zu der Kolbenstange übertragen. Die Führungsbahn kann ein um die Kolbenstange verlaufender Kurvenbogen sein, an dem das Eingriffsglied bei einer zwischen den Kurvenglied und dem Eingriffsglied um die Kolbenstange stattfindenden Drehbewegung von der Kolbenstange abgehoben wird. Vorzugsweise verläuft die Führungsbahn jedoch in Längsrichtung der Kolbenstange und weist zu der Kolbenstange, d.h. zu der Längsrichtung der Kolbenstange, eine Neigung auf. Obgleich die Führungsbahn auch in dieser Ausbildung gekrümmt sein kann, weist die Neigung jedoch vorzugsweise einen konstanten Neigungswinkel auf. Besonders bevorzugt ist die Führungsbahn kontinuierlich, d.h. ohne Knickstellen und insbesondere ohne Sprünge, geneigt.

In bevorzugter Ausführung sind das Dosierglied und das Getriebeausgangsglied separate Körper, die relativ zueinander in Längsrichtung der Kolbenstange bewegbar sind. Grundsätzlich wäre es jedoch auch denkbar, das Dosierglied und das Getriebeausgangsglied einteilig auszubilden. In der bevorzugten zweiteiligen Ausbildung hintergreift das Getriebeausgangsglied das Dosierglied quer zu einer Senkrechten auf eine Längsachse der Kolbenstange, um das Dosierglied aus dem Eingriff mit der Kolbenstange herauszuziehen.

Das Rücksetz-Betätigungselement und das Getriebeeingangsglied sind in ebenfalls bevorzugter Ausführung separate Körper. Grundsätzlich gilt jedoch auch hier, das das Rücksetz-Betätigungselement und das Getriebeeingangsglied einteilig ausgeführt sein können. In der bevorzugten zweiteiligen Ausführung sind das Rücksetz-Betätigungselement und das Getriebeeingangsglied vorzugsweise so miteinander verbunden, dass sie entlang der Längsachse der Kolbenstange relativ zueinander bewegbar und/oder um die Längsachse der Kolbenstange relativ zueinander drehbar sind. Die Bewegbarkeit des Getriebeeingangsglieds relativ zu dem Rücksetz-Betätigungselement ist insbesondere dann von Vorteil, wenn das Rücksetz-Betätigungselement einen Abschnitt des Gehäuses bildet.

Besonders bevorzugt wird, wenn ein Federelement vorgesehen und so angeordnet ist, dass es durch die Einrückbewegung des Rückstell-Betätigungselements gespannt wird und bei der Ausrückbewegung aufgrund seiner Spannkraft die Relativbewegung zwischen dem Getriebeeingangsglied und dem Getriebeausgangsglied bewirkt. Dieses Federelement kann insbesondere von einer Ausgleichsfeder gebildet werden, wie sie üblicherweise verwendet wird, um ein mit dem Produkt gefülltes Behältnis in Vorschubrichtung gegen das Gehäuse auf Anschlag zu drücken. Solche Ausgleichsfedern dienen im allgemeinen dazu, Längentoleranzen zwischen einem von dem Gehäuse aufgenommenen Produktbehältnis und anderen Komponenten der Vorrichtung auszugleichen. Mit solch einer Ausgleichsfeder kann vorteilhafterweise die Relativbewegung zwischen dem Getriebeeingangsglied und dem Getriebeausgangsglied bewirkt werden. Dies ist auf einfache Weise dann möglich, wenn die Relativbewegung parallel zu der Längsachse der Kolbenstange stattfindet oder eine axiale Bewegungskomponente umfasst.

Weitere bevorzugte Merkmale werden in den Unteransprüchen beschrieben.

Nachfolgend wird die Erfindung anhand eines Ausführungsbeispiels erläutert. Es zeigen:
- Fig. 1: eine Produktausschüttvorrichtung in einem Ausgangszustand vor einer ersten Auswahl einer Produktdosis,
- Fig. 2: die Produktausschüttvorrichtung in einem Endzustand, in dem eine Kolbenstange der Vorrichtung eine vorderste Position einnimmt und mit einem Dosierglied in Eingriff ist,
- Fig. 3: die Produktausschüttvorrichtung in einem Zustand, in dem die Kolbenstange und das Dosierglied außer Eingriff sind und die Kolbenstange in eine hinterste Ausgangsposition zurückgesetzt worden ist,
- Fig. 4: den Querschnitt A-A der Fig. 2
- Fig. 5: den Querschnitt B-B der Fig. 3
- Fig. 6: die Kolbenstange, das Dosierglied und Getriebeglieder eines Kurvengetriebes in einem dreidimensionalen Schnitt, wobei die Kolbenstange und das Dosierglied in Eingriff sind,
- Fig. 7: die Kolbenstange, das Dosierglied und die Getriebeglieder in einem dreidimensionalen Schnitt, wobei die Kolbenstange und das Dosierglied außer Eingriff sind,
- Fig. 8: Komponenten der Vorrichtung in dreidimensionaler Darstellung,
- Fig. 9: eine Dosisanzeigehülse und mit der Dosisanzeigehülse zusammenwirkende Komponenten in dreidimensionaler Darstellung und
- Fig. 10: die Dosisanzeigehülse in mehreren Darstellungen.

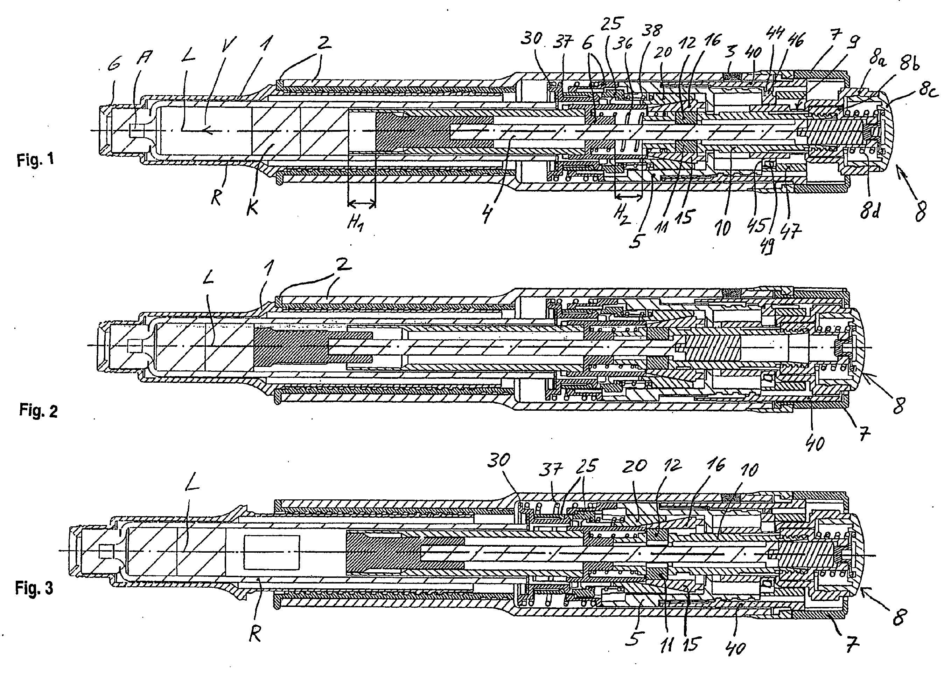

Fig. 1 zeigt eine Vorrichtung für die Ausschüttung einer ausgewählten Dosis eines flüssigen Produkts. Das Produkt kann insbesondere ein Medikament sein, beispielsweise Insulin.

Die Vorrichtung weist ein zweiteiliges Gehäuse mit einem ersten, vorderen Gehäuseabschnitt 1 und einem zweiten, hinteren Gehäuseabschnitt 2 auf, die je als Hülsenteil geformt sind. Die beiden Gehäuseabschnitte 1 und 2 sind miteinander verschraubt. Der vordere Gehäuseabschnitt 1 enthält ein mit dem Produkt gefülltes Reservoir R. Das Reservoir R wird von einem austauschbaren Behältnis, im Ausführungsbeispiel von einer hohlzylindrischen Ampulle gebildet. Das Behältnis wird von hinten in den vorderen Gehäuseabschnitt 1 bis gegen einen von dem vorderen Gehäuseabschnitt 1 gebildeten Anschlag eingeschoben. Das Behältnis weist an seinem vorderen Ende einen Auslass A auf, der von einem Septum steril abschlossen wird. Um das Produkt aus dem derart gebildeten Reservoir R ausschütten zu können wird eine Kanüle durch das Septum bis in den Auslass A eingeführt. An seinem hinteren Ende ist das Reservoir R von einem Kolben K dicht verschlossen. Durch Vorschub des Kolbens K entlang einer Längsachse L in eine Vorschubrichtung V auf den Auslass A zu wird Produkt aus dem Reservoir R verdrängt, d.h. durch den Auslass A ausgeschüttet.

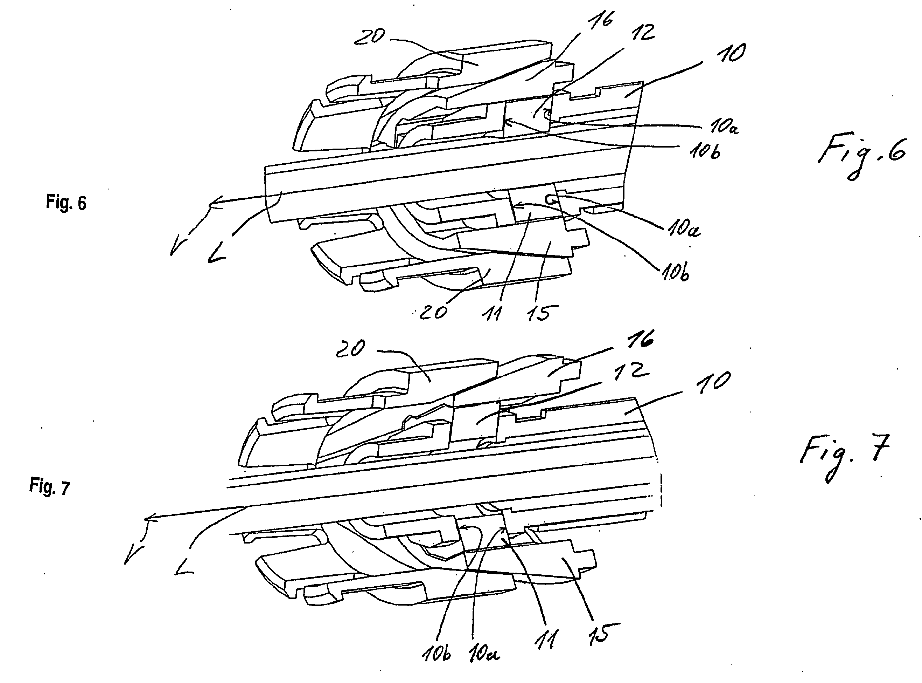

Die Bewegung des Kolbens K in die Vorschubrichtung V wird mittels einer Kolbenstange 4 bewirkt, die von dem Gehäuse 1/2 entlang der Längsachse L, d.h. axial, geradgeführt wird. Die Kolbenstange 4 ist mit einer Dosier- und Betätigungseinrichtung gekoppelt. Die Dosier- und Betätigungseinrichtung wird gebildet von einer Dosierhülse 7, einem hülsenförmigen Dosier- und Betätigungsknopf 8 und einem hülsenförmigen Übertragungselement 10. Mit dieser Dosier- und Betätigungseinrichtung 7/8/10 ist ein geteiltes Dosierglied 11/12 verbunden. Das Dosierglied 11/12 ist axial in zwei separate, gleiche Teile geteilt. Die beiden Teile werden im folgenden als erstes Dosierglied 11 und als zweites Dosierglied 12 bezeichnet. Das erste Dosierglied 11 und das zweite Dosierglied 12 sind mit der als Gewindestange ausgebildeten Kolbenstange 4 je in einem Gewindeeingriff um die Längsachse L, die gleichzeitig auch die Längsachse der Kolbenstange 4 ist. Das erste Dosierglied 11 und das zweite Dosierglied 12 sind mit dem Übertragungselement 10 axial verschiebegesichert und in Bezug auf Verdrehungen um die Längsachse L verdrehgesichert verbunden. Allerdings ist sowohl das erste Dosierglied 11 als auch das zweite Dosierglied 12 relativ zu dem Dosierelement 10 und insbesondere relativ zu der Kolbenstange 4 in eine Richtung quer zu der Längsachse L bewegbar. Für die Querbewegbarkeit werden das erste Dosierglied 11 und das zweite Dosierglied 12 von dem Übertragungselement 10 geradgeführt, indem das Übertragungselement 10 für die beiden Dosierglieder 11 und 12 je einen quer zu der Längsachse L weisenden Führungsschacht bildet, der gleichzeitig auch als axiale Verschiebesicherung und Verdrehsicherung wirkt. Wie besser beispielsweise anhand der Figuren 6 und 7 erkennbar ist, weisen die Führungsschächte je eine in die Vorschubrichtung V weisende Seitenwand 10a und eine gegen die Vorschubrichtung V weisende Seitenwand 10b auf. Die Seitenwände 10a und 10b bilden je eine quer zu der Längsachse weisende, gerade Querführungsbahn für eines der Dosierglieder 11 und 12. Zwischen den beiden Querführungsbahnen 10a und 10b eines Führungsschachts ist je eines der Dosierglieder 11 und 12 eingefasst und gleitgeführt, so dass die beiden von den Paaren der Querführungsbahnen 10a und 10b gebildeten Querführungen nur Gleitbewegungen exakt senkrecht zu der Längsachse L zulassen. Die Dosierglieder 11 und 12 bilden selbst die geführten Eingriffsgleitstücke. Die Querführungsbahnen 10a und 10b bilden in Verbindung mit den Dosiergliedern 11 und 12 einen Teil eines Kurvengetriebes.

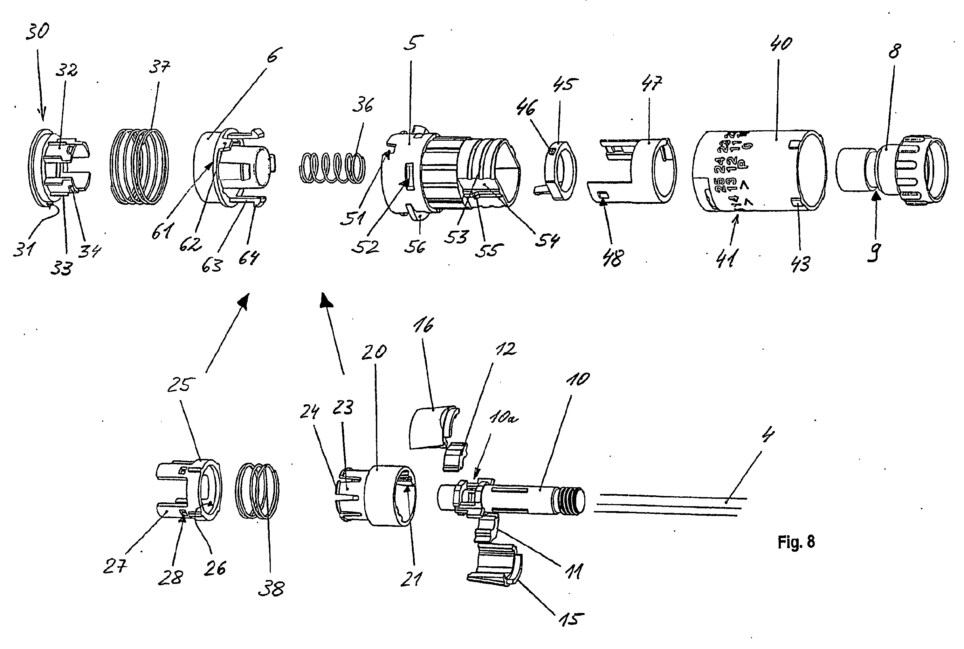

In Bezug auf die Form, die Funktion und das Zusammenwirken der für die Dosierung und Ausschüttung des Produkts zusammenwirkenden Komponenten sei ergänzend stets auf die Fig. 8 verwiesen.

Der Dosier- und Betätigungsknopf 8 ist mit dem Übertragungselement 10- lösbar verbunden. Der Dosier- und Betätigungsknopf 8 ist aus mehreren Einzelteilen zusammengesetzt, nämlich einem aus dem hinteren Gehäuseabschnitt 2 herausragenden äußeren Hülsenteil 8a, einem eingesetzten inneren Hülsenteil 8b, einem von hinten in das äußere Hülsenteil 8a eingesteckten und verrasteten Endknopf 8c und einer zwischen dem Endknopf 8c und dem inneren Hülsenteil 8b axial gespannten Feder 8d. Das innere Hülsenteil 8b dient der Befestigung des Dosier- und Betätigungsknopfs 8 an dem Übertragungselement 10 und wird deshalb im Folgenden auch als Befestigungsteil 8b bezeichnet. Das äußere Hülsenteil 8a ist für den Verwender für die Betätigung des Verabreichungsgeräts zugänglich und wird deshalb im Folgenden auch als Betätigungsteil 8a bezeichnet. Das Betätigungsteil 8a ist mit dem Endknopf 8c verschiebegesichert verbunden und über die auf Druck gespannte Feder 8d an dem Befestigungsteil 8b abgestützt. Das Betätigungsteil 8a ist relativ zu dem Befestigungsteil 8b und deshalb auch relativ zu dem Übertragungselement 10 in und gegen die Vorschubrichtung V bewegbar. Die Feder 8d drückt das Betätigungsteil 8a entgegen der Vorschubrichtung V auf Anschlag gegen das Befestigungsteil 8b, so dass es relativ zu dem Befestigungsteil 8b die in den Figuren 1-3 gezeigte hintere Anschlagposition einnimmt. Entsprechend kann es durch Druck gegen die Elastizitätskraft der Feder 8d relativ zu dem Befestigungsteil 8b und dem Übertragungselement 10 in die Vorschubrichtung V bewegt werden. Diese Bewegbarkeit in die Vorschubrichtung V ist vorteilhaft für die Betätigung der Dosier- und Betätigungseinrichtung 7/8/10, die aufgrund dieses Merkmals in sich einfedern kann. Die Komponenten 8a bis 8d werden im folgenden in ihrer Gesamtheit als Dosier- und Betätigungsknopf 8 bezeichnet.

Zu erwähnen ist noch, dass an der Außenmantelfläche des Dosier- und Betätigungsknopfs 8, im Ausführungsbeispiel an der Außenmantelfläche des äußeren Hülsenteils 8a, in einem das Übertragungselement 10 umgebenden Abschnitt eine umlaufende Nut 9 geformt ist. Diese Nut 9 wirkt mit einem als Ring gebildeten Sperrglied 45 zusammen, das die Außenmantelfläche des Dosier- und Betätigungsknopfs 8 umgibt und mittels einem Federelement 49 nach radial einwärts gegen die Außenmantelfläche des Dosier- und Betätigungsknopfs 8 gespannt wird.

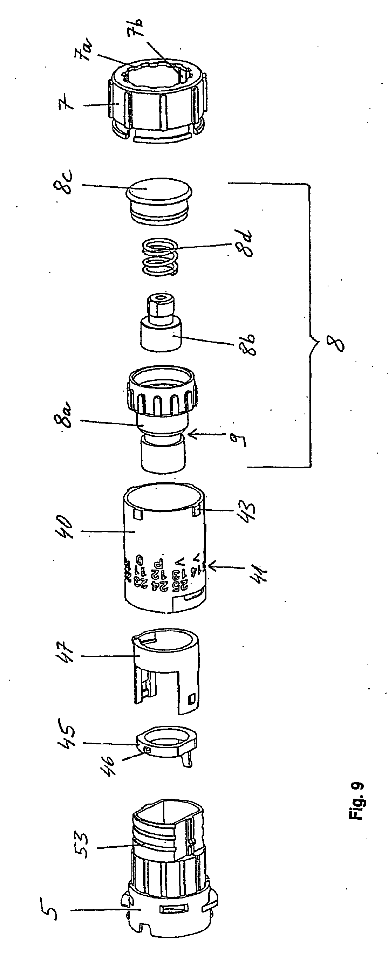

Die Dosierhülse 7 ist an dem hinteren Ende des hinteren Gehäuseabschnitts 2 angebracht und um die Längsachse L relativ zu dem hinteren Gehäuseabschnitt 2 drehbar. Die Dosierhülse 7 bildet an ihrem hinteren Ende einen nach radial innen ragenden Kragen, der über seinen Innenumfang gleichmäßig verteilt Zacken 7a bildet (Fig. 9). An einer den Zacken 7a gegenüberliegenden Außenmantelfläche bildet der Dosier- und Betätigungsknopf 8 in entsprechender Teilung gerade, axiale Führungsnuten, in denen je einer der Zacken 7a axial geradgeführt wird. Auf diese Weise sind die Dosierhülse 7 und der Dosier- und Betätigungsknopf 8 um die Längsachse L verdrehgesichert, aber relativ zueinander axial beweglich verbunden.

Ein Kolbenstangenhalter 6 bildet eine axiale Geradführung für die Kolbenstange 4. Der Kolbenstangenhalter 6 ist mit dem hinteren Gehäuseabschnitt 2 nicht bewegbar verbunden, insbesondere ist der Kolbenstangenhalter 6 relativ zu dem hinteren Gehäuseabschnitt 2 weder axial bewegbar noch um die Längsachse L verdrehbar. Zwischen dem Kolbenstangenhalter 6 und der Kolbenstange 4 sind ausschließlich axiale Relativbewegungen möglich. Auf diese Weise ist die Kolbenstange 4 relativ zu dem hinteren Gehäuseabschnitt 2 axial geradgeführt.

Radial über dem ersten Dosierglied 11 ist ein Gleitstück 15 und radial über dem zweiten Dosierglied 12 ist ein weiteres Gleitstück 16 je axial verschiebegesichert, aber radial geradgeführt und in diesem Sinne radial bewegbar zwischen einem rückwärtigen Ende des Kolbenstangenhalters 6 und einem radial nach innen ragenden Kragen eines Mechanikhalters 5 gelagert. Die Gleitstücke 15 und 16 haben die gleiche Form und erfüllen die gleiche Funktion je in Bezug auf das zugeordnete erste Dosierglied 11 und zweite Dosierglied 12. Die Anordnung der Gleitstücke 15 und 16 ist symmetrisch zu der Längsachse L. Der Mechanikhalter 5 ist mit dem hinteren Gehäuseabschnitt 2 unbeweglich verbunden, insbesondere ist er relativ zu dem hinteren Gehäuseabschnitt 2 axial nicht bewegbar, wofür die Dreiviertel-Rippe 56 als Anschlag dient, und um die Längsachse L nicht verdrehbar.

Die Gleitstücke 15 und 16 bilden weitere Getriebeglieder des Kurvengetriebes. Da sie je mit einem der Dosierglieder 11 und 12 in einem unmittelbaren Eingriff stehen, werden sie im Folgenden als die Getriebeausgangsglieder des Kurvengetriebes bezeichnet. Sie wirken je mit einem Getriebeeingangsglied 20 zusammen, das als Gleithülse ausgebildet ist und im Folgenden unter dieser Bezeichnung in Bezug genommen wird. Die Gleithülse 20 bildet ein weiteres Kurvenglied und die beiden Gleitstücke 15 und 16 bilden je ein Eingriffsglied des Kurvengetriebes. Die Gleithülse 20 ist relativ zu dem ersten Dosierglied 11 und zweiten Dosierglied 12, den beiden Gleitstücken 15 und 16 und relativ zu dem hinteren Gehäuseabschnitt 2 axial bewegbar. Relativ zu dem hinteren Gehäuseabschnitt 2 ist sie ferner um die Längsachse L verdrehbar. Der Mechanikhalter 5 bildet mit einer kreiszylindrischen Innenmantelfläche ein Drehgleitlager und eine axiale Geradführung für die Gleithülse 20.

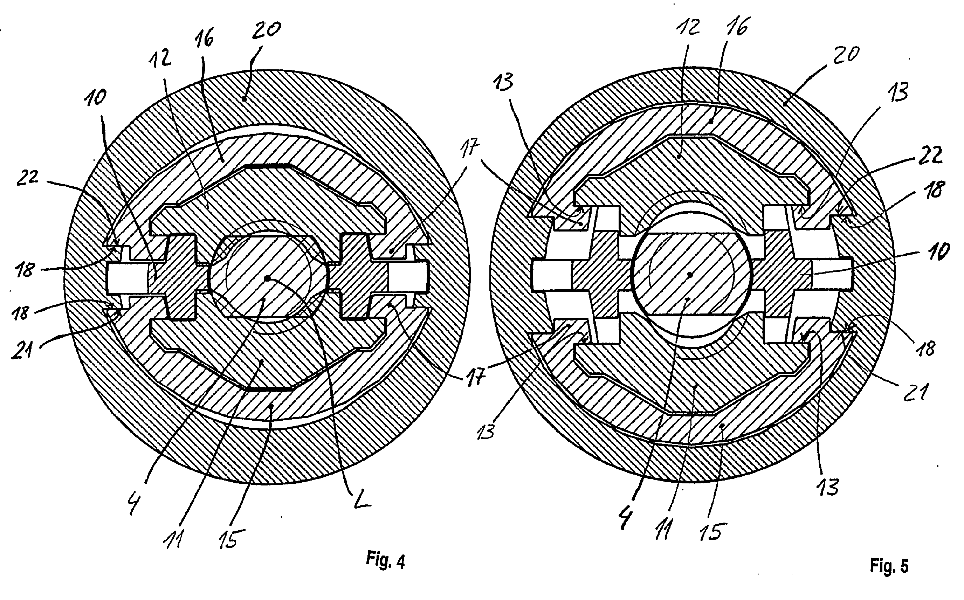

Die Fig. 4 und 5 zeigen das Kurvengetriebe mit dem geteilten Dosierglied 11/12 und der Kolbenstange 4 je im gleichen Querschnitt, bezogen auf das Dosierglied 11/12, allerdings in unterschiedlichen Getriebezuständen. Die gleichen Getriebezustände sind in den Fig. 6 und 7 je in eine dreidimensionalen Schnitt dargestellt. Was die Wirkungsweise des Kurvengetriebes anbetrifft, so sei im folgenden stets auch ergänzend auf die Figuren 4 bis 7 verwiesen.

Die Gleitstücke 15 und 16 sind an ihrem Außenmantel je konisch. Die Gleithülse 20 bildet an ihrem Innenmantel, der den Konusflächen der Gleitstücke 15 und 16 zugewandt ist, einen entsprechenden Gegenkonus. Die Konusaussenflächen der Gleitstücke 15 und 16 und die Fläche des Gegenkonus der Gleithülse 20 sind zueinander parallel unter Ausbildung eines über die gesamten Konusflächen gleichmäßig dicken Konusspalts.

Wie am besten in den Fig. 4 und 5 erkennbar, bildet die Gleithülse 20 zwei Führungsbahnen 21 für das Gleitstück 15 und zwei Führungsbahnen 22 für das Gleitstück 16. Die Gleitstücke 15 und 16 bilden entsprechende Eingriffsbahnen 18, die mit den Führungsbahnen 21 und 22 Gleitkontakt haben. Die Führungsbahnen 21 sind an der Innenmantelfläche der Gleithülse 20 beidseits der Längsachse L einander diametral gegenüberliegend gebildet. Das Gleiche gilt für die Führungsbahnen 22. Die Führungsbahnen 21 und 22 erstrecken sich in axialer Richtung und weisen gegenüber der Längsachse L je eine konstant Neigung auf, d.h. die Führungsbahnen 21 und 22 sind gerade. Die Führungsbahnen 21 sind zueinander parallel. Auch die Führungsbahnen 22 sind zueinander parallel. Die im Querschnitt der Figuren 4 und 5 an der linken und rechten Seite der Gleithülse 20 gebildeten Führungsbahnen 21 und 22 laufen im Längsschnitt der Fig. 1 gesehen in Vorschubrichtung V unter einem spitzen Winkel, den eine zu der Längsachse L parallele Gerade hälftig teilt, pfeilförmig aufeinander zu. Bei einer Axialbewegung der Gleithülse 20 relativ zu den Gleitstücken 15 und 16 werden die Gleitstücke 15 und 16 somit über ihre Eingriffsbahnen 18 an den Führungsbahnen 21 und 22 so geführt, das die Gleitstücke 15 und 16 quer zu der Längsachse L, im Ausführungsbeispiel exakt senkrecht zu der Längsachse L, voneinander weg oder aufeinander zu bewegt werden.

Das erste Dosierglied 11 ist in dem Gleitstück 15 axial geradgeführt aufgenommen. Entsprechend ist das zweite Dosierglied 12 axial geradgeführt in seinem Gleitstück 16 aufgenommen. Die Gleitstücke 15 und 16 weisen an ihren einander zugewandten Unterseiten im Querschnitt linksseitig und rechtsseitig nach innen aufeinander zu ragende Stege 17 auf. Das Gleitstück 15 hintergreift mit seinen beiden Stegen 17 das erste Dosierglied 11, und das Gleitstück 16 hintergreift mit seinen beiden Stegen 17 das zweite Dosierglied 12. Werden die beiden Gleitstücke 15 und 16 quer zur Längsachse L voneinander wegbewegt, so werden durch die hintergreifenden Stege 17 die beiden Dosierglieder 11 und 12 in gleicher Weise quer zu der Längsachse L voneinander weg und dadurch aus dem Eingriff mit der Kolbenstange 4 bewegt. Bei einer Querbewegung der Gleitstücke 15 und 16 aufeinander zu drücken die Gleitstücke 15 und 16 über ihre Innenmantelflächen auch wieder die Dosierglieder 11 und 12 auf einander zu und dadurch wieder in den Eingriff mit der Kolbenstange 4. Wie bereits erwähnt, sind die Dosierglieder 11 und 12 in Ihrem jeweiligen Gleitstück 15 und 16 axial geradverschiebbar geführt. Die Stege 17 bilden für die Geradführung Führungsbahnen, und die Dosierglieder 11 und 12 bilden daran geführte Eingriffsbahnen 13, die von den Stegen 17 hintergriffen werden.

Der in dem Zustand der Fig. 1 bestehende Gewindeeingriff zwischen der Kolbenstange 4 und dem geteilten Dosierglied 11/12 wird durch eine zwischen den Gehäuseabschnitten 1 und 2 stattfindende axiale Relativbewegung gelöst. Diese Relativbewegung findet bei einem Auseinanderschrauben der Gehäuseabschnitte 1 und 2 statt. Bei dem Auseinanderschrauben wird zunächst das von den Gehäuseabschnitten 1 und 2 gemeinsam gebildete Gehäuse verlängert, was einer Relativbewegung zwischen den Gehäuseabschnitten 1 und 2 in axialer Richtung entspricht. Es wird somit die für beispielsweise einen Austausch des Reservoirs R erforderliche Relativbewegung zwischen den Gehäuseabschnitten 1 und 2 benutzt, um den Eingriff zwischen der Kolbenstange 4 und dem geteilten Dosierglied 11/12 und die durch diesen Eingriff erhaltene axiale Verschiebesicherung zwischen der Kolbenstange 4 und der Dosier- und Betätigungseinrichtung 7/8/10 zu lösen.

Um die axiale Relativbewegung zwischen den Gehäuseabschnitten 1 und 2 zum Lösen des Eingriffs nutzen zu können, ist die Gleithülse 20 mit dem vorderen Gehäuseabschnitt 1 verbunden, und zwar derart, das sie dessen axiale Bewegung relativ zu dem hinteren Gehäuseabschnitt mitmacht, während die Gleitstücke 15 und 16 ihre Axialposition relativ zu dem hinteren Gehäuseabschnitt 2 beibehalten. Für die axiale Verschiebesicherung der Gleitstücke 15 und 16 sorgt, wie gesagt, deren Lagerung zwischen dem Mechanikhalter 5 und dem Kolbenstangenhalter 6. Die Kopplung der Gleithülle 20 an den vorderen Gehäuseabschnitt 1 bildet eine Reservoirhaltefeder 37, die im vollkommen zusammengeschraubten Zustand der beiden Gehäuseabschnitte 1 und 2 axial zwischen dem Kolbenstangenhalter 6 und einem Reservoirhalter 30 auf Druck gespannt ist. Hierfür ist die Reservoirhaltefeder 37 zwischen dem Kolbenstangenhalter 6 und dem Reservoirhalter 30 gespannt. Die Reservoirhaltefeder 37 drückt den Reservoirhalter 30 gegen den rückwärtigen Rand des Behältnisses, das das Reservoir R bildet. Die Reservoirhaltefeder 37 drückt das Behältnis gegen eine in dem vorderen Gehäuseabschnitt 1 gebildete Anschlagfläche. Die Anordnung solch einer Reservoirhaltefeder 37 ist üblich, um Längentoleranzen des Behältnisses, der Gehäuseabschnitte 1, 2 und den Komponenten auszugleichen, an denen das Behältnis in Längsrichtung abgestützt ist.

An dem Reservoirhalter 30 ist ein Mitnehmer 25 verschiebe- und verdrehgesichert befestigt. Der Mitnehmer 25 bildet ein Übertragungsglied zwischen dem Reservoirhalter 30 und der Gleithülse 20, wenn der Reservoirhalter 30 aufgrund der Federkraft der Reservoirhaltefeder 37 einer axialen Bewegung des vorderen Gehäuseabschnitts 1 folgt. Die Gleithülse 20 und der Mitnehmer 25 hintergreifen einander derart, dass die Gleithülse 20 bei der Axialbewegung von dem Mitnehmer 25 mitgenommen wird. Im Ausführungsbeispiel ist zwischen der Gleithülse 20 und dem Mitnehmer 25 eine Toleranzausgleichsfeder 38 in axialer Richtung gespannt. Grundsätzlich könnten die Gleithülse 20 und der Mitnehmer 25 jedoch auch in Bezug auf die axiale Richtung starr miteinander verbunden sein. Allerdings ist zwischen der Gleithülse 20 und dem Mitnehmer 25 eine Drehbewegung um die Längsachse L möglich. Insofern ist zwischen der Gleithülse 20 und dem Mitnehmer 25 das Drehgelenk zwischen der Dosier- und Betätigungseinrichtung 7/8/10 und dem hinteren Gehäuseabschnitt 2 gebildet.

Der Zusammenbau der Getriebekomponenten 20, 25 und 30 und deren Anordnung relativ zu dem Kolbenstangenhalter 6 ist am besten in Fig. 8 erkennbar. So sind insbesondere die einander zugewandten Schultern 31 und 61 des Reservoirhalters 30 und des Kolbenstangenhalters 6 erkennbar, zwischen denen die Reservoirhaltefeder 37 gespannt ist. Der Reservoirhalter 30 weist ein kreiszylindrisches, vorderes Hülsenteil mit der Schulter 31 auf. Von dem Hülsenteil ragen rückwärtig mehrere Schuhe 32 ab. Am hinteren Ende von jedem der Schuhe 32 ist ein nach radial außen ragender Rastnocken 34 geformt. Ferner weist jeder der Schuhe 32 Seitenführungen 33 auf. Der Reservoirhalter 30 ist mit seinen Schuhen 32 zwischen einem in radialer Richtung äußeren und einem mittleren Hülsenteil des Kolbenstangenhalters 6 einschiebbar. Hierfür weist der Kolbenstangenhalter 6 in einem Hülsenboden den Schuhen 32 entsprechende Aussparungen auf. Der Kolbenstangenhalter 6 und der Reservoirhalter 30 sind über die Schuhe 32 und die Aussparungen verdrehgesichert miteinander verbunden. Von der rückwärtigen Seite wird der Mitnehmer 25 über das mittlere Hülsenteil des Kolbenstangenhalter 6 auf den Reservoirhalter 30 aufgeschoben. Von einem hinteren Hülsenteil des Mitnehmers 25 ragen Zungen 27 in Anzahl und Anordnung den Schuhen 32 entsprechend ab. Je eine der Zungen 27 wird auf je einen der Schuhe 32 aufgeschoben und mittels der Rastnocken 34 verrastet, wobei die Rastnocken 34 in entsprechend an den Zungen 27 gebildete Ausnehmungen 28 einrasten. Die Verbindung zwischen dem Mitnehmer 25 und dem Reservoirhalter 30 kann als vollkommen starr betrachtet werden.

Die Gleithülse 20 weist ein rückwärtiges Hülsenteil und eine Mehrzahl von biegeelastischen Zungen 23 auf, die von dem Hülsenteil in Vorschubrichtung V ab und auf den Mitnehmer 25 zu ragen. An den vorderen Enden der Zungen 23 sind Mitnehmernocken 24 gebildet, die von den Zungen 23 nach radial außen abragen. Die Mitnahme der Gleithülse 20 bei einer Axialbewegung des Mitnehmer 25 wird durch einen Eingriff bewirkt, der im verbundenen Zustand zwischen den Mitnehmernocken 24 und einer Mitnehmerschulter 26 besteht, die an dem rückwärtigen Ende des Mitnehmers 25 nach radial innen vorragt und von den Mitnehmernocken 24 im verbundenen Zustand hintergriffen wird. Gleichzeitig ermöglicht der Eingriff Relativdrehbewegungen um die Längsachse L.

Ferner ist in Fig. 8 auch die verschiebe- und verdrehgesicherte Verbindung zwischen dem Mechanikhalter 5 und dem Kolbenstangenhalter 6 erkennbar. Von dem Hülsenteil des Kolbenstangenhalters 6 ragen rückwärtig biegeelastische Zungen 63 mit radial nach außen vorragenden Rastnocken 64 ab. Mit den Rastnocken 64 verrastet der Kolbenstangenhalter 6 in entsprechenden Ausnehmungen 52 des Mechanikhalters 5. Eine Verdrehsicherung zwischen dem Mechanikhalter 5 und dem Kolbenstangenhalter 6 wird ferner durch Eingriff eines von dem Mechanikhalter 5 axial abragenden Führungsvorsprungs 62 in eine Führungsausnehmung 51 des Mechanikhalters 5 erhalten. Über die Rastverbindung durch die Rastnocken 64 und die Führung durch den Führungsvorsprung 62 sind der Mechanikhalter 5 und der Kolbenstangenhalter 6 verschiebe- und verdrehgesichert miteinander verbunden. Der Mechanikhalter 5 ist mit dem hinteren Gehäuseabschnitt 2 verschiebe- und verdrehgesichert verbunden, so das gleiches auch für den Kolbenstangenhalter 6 gilt.

Zwischen dem Kolbenstangenhalter 6 und dem Übertragungselement 10 der Dosier- und Betätigungseinrichtung 7/8/10 ist eine Rückstellfeder 36 axial gespannt. Die Rückstellfeder 36 dient der Rückführung der Dosier- und Betätigungseinrichtung 7/8/10, des geteilten Dosierglieds 11/12 und der Kolbenstange 4 nach einer Produktausschüttung.

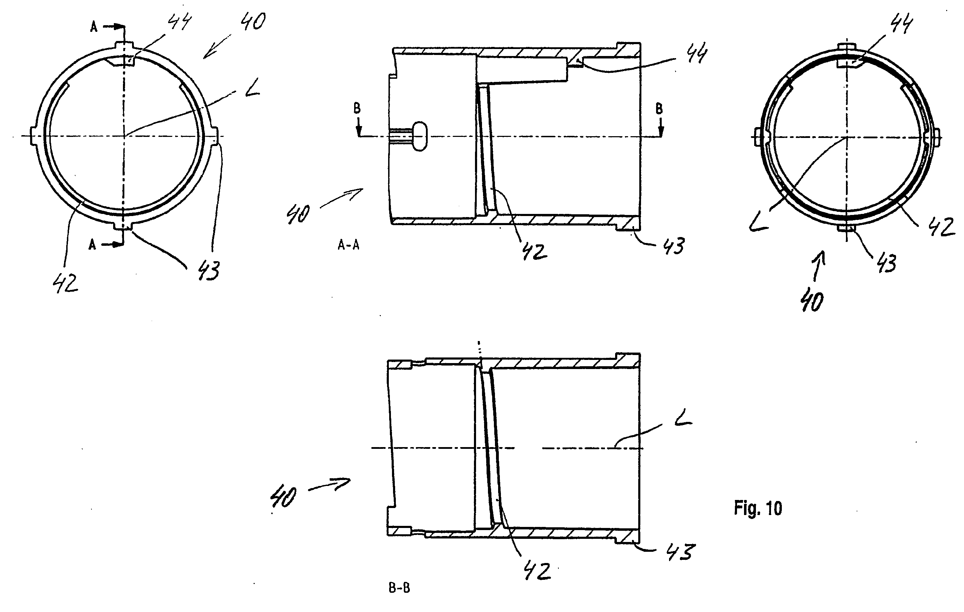

Die Vorrichtung umfasst ferner eine Dosisanzeigehülse 40, die an Ihrer Außenmantelfläche mit einer Dosisskala 41 versehen ist. Die Dosisanzeigehülse 40 ist in den Figuren 8 und 9 je in einer dreidimensionalen Ansicht von der Seite und in Figur 10 in zwei Längsschnitten, einer Frontansicht und einer Rückansicht dargestellt. Sie ist mit dem Mechanikhalter 5 in einem Gewindeeingriff. Der Gewindeeingriff besteht zwischen einem Außengewinde 53 in einem rückwärtigen Abschnitt des Mechanikhalters 5 und einem Innengewinde 42 der Dosisanzeigehülse 40. Die Gewindeachse der beiden Gewinde 42 und 53 fällt mit der Längsachse L zusammen. Die Dosisskala 41 wird von Dosisangaben gebildet, die an der Außenmantelfläche der Dosisanzeigehülse 40 spiralig umlaufend angeordnet sind. Im Ausführungsbeispiel bilden den auswählbaren Dosiseinheiten entsprechende Zahlen die Dosisangaben. Die Dosisanzeigehülse 40 ist mit der Dosierhülse 7 axial geradverschiebbar aber im Bezug auf die Längsachse L verdrehgesichert verbunden. Zu diesem Zweck weist die Dosisanzeigehülse 40 an Ihrem hinteren Ende eine Mehrzahl von radial abragenden Führungsnocken 43 auf, die in entsprechende Führungsnuten 7b (Fig. 9) an der Innenmantelfläche der Dosierhülse 7 hineinragen und darin axial geradgeführt sind. Bei einer Drehbewegung der Dosierhülse 7 wird aufgrund dieses Eingriffs auch die Dosisanzeigehülse 40 um die Längsachse L verdreht. Wegen des Gewindeeingriffs mit dem Mechanikhalter 5 schraubt sich die Dosisanzeigehülse 40 somit bei der durch die Drehbewegung der Dosierhülse 7 vorgenommenen Dosisauswahl relativ zu dem Mechanikhalter 5 und damit auch relativ zu dem hinteren Gehäuseabschnitt 2 gegen die Vorschubrichtung V nach hinten.
Die Dosisanzeigehülse 40 ragt mit ihrem das Innengewinde 42 und die Dosisskala 41 bildenden Abschnitt in einen Ringspalt, der zwischen dem hinteren Gehäuseabschnitt 2 und dem Mechanikhalter 5 verbleibt. In dem Abschnitt, der der Dosisskala 41 radial außen gegenüber liegt, weist der hintere Gehäuseabschnitt 2 ein Fenster 3 auf, durch das die Dosisskala 41 abgelesen werden kann. Die Steigung des Innengewindes 42 entspricht der Steigung der spiraligen Dosisskala 41.

Radial einwärts von einem Längsabschnitt der Dosisanzeigehülse 40, der über den Mechanikhalter 5 nach hinten hinaus ragt, ist das bereits erwähnte Sperrglied 45 angeordnet, das seiner Form wegen im Folgenden als Sperrring bezeichnet wird. Der Sperrring 45 ist in einem Hülsenteil eines Sperrringhalters 47 verdrehgesichert und axial verschiebegesichert gelagert. Der Sperrringhalter 47 ist an dem Mechanikhalter 5 verdreh- und verschiebegesichert befestigt, und zwar über eine Führungsfläche 54 des Mechanikhalters 5 und eine Rastverbindung, die zwischen einem Rastnocken 55 des Mechanikhalters 5 und einer Ausnehmung 48 des Sperrringhalters 47 gebildet wird (Fig. 8).

Der Sperrring 45 bildet einen Resetgegennocken 46, der von der äußeren Mantelfläche des Sperrrings 45 nach radial außen auf die Dosisanzeigehülse 40 zu vorragt. Von der zugewandten Innenmantelfläche der Dosisanzeigehülse 40 ragt nach radial einwärts ein Resetnocken 44 ab (Fig. 10). Der Resetnocken 44 ist in Bezug auf die Dosisskala 41 in solch einer Drehwinkelposition gebildet, dass er genau dann in der radialen Flucht mit dem Resetgegennocken 46 ist und radial gegen den Nocken 46 drückt, wenn die Dosisanzeigehülse 40 relativ zu dem hinteren Gehäuseabschnitt 2 eine Nulldosis-Position einnimmt, in der die der Nulldosis entsprechende Dosisangabe in dem Fenster 3 angezeigt wird. Nimmt die Dosisanzeighülse 40 in Bezug auf den Mechanikhalter 5 und den hinteren Gehäuseabschnitt 2 eine Position ein, die nicht der Nulldosis entspricht, so ist der Resetgegennocken 46 von dem Resetnocken 44 frei, d.h. es verbleibt ein radial lichter Abstand zwischen dem Resetnocken 45 und der zugewandten Innenmantelfläche der Dosisanzeigehülse 40.

Der Sperrring 45 ist relativ zu dem Sperrringhalter 47 radial zu der Längsachse L geradbewegbar. Dem Resetgegennocken 46 in Bezug auf die Längsachse L diametral gegenüber ist zwischen dem Sperrring 45 und dem Sperrringhalter 47 das Federelement 49 angeordnet, das den Sperring 45 gegen die Außenmantelfläche des Dosier- und Betätigungsknopfs 8 drückt. Das Federelement 49 wirkt als Druckfeder.

Die Funktionsweise der Vorrichtung wird nachfolgend anhand der Figuren 1 bis 3 beschrieben, in denen die Vorrichtung je in einem anderen Zustand dargestellt ist. Auf die weiteren Figuren sei ergänzend stets hingewiesen.

In Fig. 1 nimmt die Vorrichtung einen Ausgangszustand ein, in dem das Reservoir R vollständig mit dem Produkt gefüllt ist und aus dem heraus die Dosisauswahl vorgenommen werden kann. Die Dosisanzeigehülse 40 nimmt ihre der Nulldosis entsprechende Position ein, d.h. durch das Fenster 3 ist die der Nulldosis entsprechende Dosisangabe ablesbar. Die Dosier- und Betätigungseinrichtung 7/8/10 nimmt zusammen mit der Kolbenstange 4 ihre hinterste Position ein, in der die Dosis ausgewählt wird. In dieser Dosisauswahlposition verbleibt zwischen der Kolbenstange 4 und dem Kolben K ein axialer, lichter Abstand H₁. Ein gleichgroßer axialer, lichter Abstand H₂ verbleibt zwischen zwei axial einander zugewandten Anschlagflächen, von denen die eine von dem Kolbenstangenhalter 6 und die andere von dem Übertragungselement 10 gebildet wird und die eine vordere Endposition für das Übertragungselement 10 und die Kolbenstange 4 definieren. Der axiale, lichte Abstand H₂ zwischen diesem Anschlagflächenpaar ist der maximale Hub der Kolbenstange 4. Durch die Dosisauswahl wird der axiale, lichte Abstand H₁ zwischen dem Kolben K und der Kolbenstange 4 verkleinert. Wird anschließend im Rahmen der Ausschüttbewegung die Kolbenstange 4 in Vorschubrichtung V um den stets gleich langen Hub H₂ bis in ihre vordere Endposition bewegt, so entspricht die bei der Dosisauswahl vorgenommene Verkleinerung des lichten Abstands H₁ zwischen der Kolbenstange 4 und dem Kolben K der ausgeschütteten Produktdosis.

Das geteilte Dosierglied 11/12 ist über die Dosier- und Betätigungseinrichtung 7/8/10 so mit der Dosisanzeigehülse 40 gekoppelt, dass der axiale Abstand H₁ zwischen dem Kolben K und der Kolbenstange 4 maximal ist, d.h. H₁ = H₂, wenn die Dosisanzeigehülse 40 ihre Nulldosis-Position einnimmt.

Zur Auswahl der Dosis wird die Dosierhülse 7 relativ zu dem hinteren Gehäuseabschnitt 2 um die Längsachse L gedreht. Hierbei wird der Dosier- und Betätigungsknopf 8 aufgrund des verdrehgesicherten Eingriffs mit der Dosierhülse 7 mitgedreht. Wegen der verdrehgesicherten Verbindungen drehen das Übertragungselement 10 und damit zusammen zwangsweise auch das geteilte Dosierglied 11/12 mit. Da die Kolbenstange 4 von dem Kolbenstangenhalter 6 axial geradgeführt wird, bewirkt die Drehbewegung des geteilten Dosierglieds 11/12 über den Gewindeeingriff eine in Vorschubrichtung V gerichtete Dosierbewegung der Kolbenstange 4. Hierdurch wird der lichte Abstand H₁ zwischen der Kolbenstange 4 und dem Kolben K um eine der ausgewählten Produktdosis entsprechende Länge verkürzt. Der lichte Abstand H₂ verändert sich hierbei nicht.

Die Dosisanzeigehülse 40 wird wegen des verdrehgesicherten Eingriffs bei der Dosierdrehbewegung von der Dosierhülse 7 mitgenommen und relativ zu dem Mechanikhalter 5 um die Längsachse L verdreht. Wegen des Gewindeeingriffs zwischen dem Mechanikhalter 5 und der Dosisanzeigehülse 40 ist der Drehbewegung der Dosisanzeigehülse 40 eine Axialbewegung gegen die Vorschubrichtung V überlagert. Nur am Rande sei erwähnt, dass die Steigung der im Eingriff befindlichen Gewinde 42 und 53 der Dosisanzeigehülse 40 und des Mechanikhalters 5 größer ist als die Steigung der im Eingriff befindlichen Gewinde der Kolbenstange 4 und des geteilten Dosierglieds 11/12. Entsprechend ist der axiale Weg, den die Dosisanzeigehülse 40 pro Umdrehung zurücklegt größer als der axiale Weg, um den die Kolbenstange 4 pro Umdrehung des geteilten Dosierglieds 11/12 bewegt wird. Dies kommt der Ablesbarkeit der Dosisskala 41 zugute. Sobald die Dosisanzeigehülse 40 relativ zu dem Mechanikhalter 5 um wenigstens eine Dosiseinheit aus ihrer Nulldosis-Position bewegt wurde, ist der Sperrring 45 von dem Resetnocken 44 frei. Die durch das Fenster 3 auf der Dosisskala 41 ablesbare Dosisangabe entspricht der axialen Länge, um die der Abstand H₁ durch die Dosierbewegung verkürzt ist.

Um die gewählte Produktdosis durch den Auslass A des Reservoirs R auszuschütten, wird der Dosier- und Betätigungsknopf 8 in die Vorschubrichtung V in den hinteren Gehäuseabschnitt 2 hineingedrückt, d.h. betätigt. Die Vorschubrichtung V ist daher auch gleichzeitig die Betätigungsrichtung des Dosier- und Betätigungsknopfs. Durch die Betätigung werden auch die mit dem Dosier- und Betätigungsknopf 8 axial verschiebegesichert verbundenen Komponeten, nämlich das Übertragungselement 10, das geteilte Dosierglied 11/12 und wegen des Gewindeeingriffs auch die Kolbenstange 4 in Vorschubrichtung um die Hublänge H₂ bewegt. Der Kolben K wird bei dieser Hubbewegung in Vorschubrichtung um eine Weglänge vorgeschoben, die der durch die Dosisauswahl vorgenommenen Verkürzung des lichten Abstands H₁ zwischen der Kolbenstange 4 und dem Kolben K im Vergleich zu dem Hub H₂ entspricht.

Fig. 2 zeigt die Vorrichtung in einem Endzustand, in dem die aus dem Reservoir R mit Hilfe der Vorrichtung maximal ausschüttbare Produktmenge nach wiederholter Verabreichung ausgeschüttet worden ist, d.h. das Reservoir R ist entleert. Der Dosier- und Betätigungsknopf 8 ist um die Hublänge H₂ in den hinteren Gehäuseabschnitt 2 hineingedrückt worden. In dieser Axialposition des Dosier- und Betätigungsknopfts 8 befindet sich dessen Nut 9 in einer radialen Flucht unter dem Sperrring 45. Die Nut 9 ist in axialer Richtung etwas breiter als der Sperrring 45, um bei einem Einfedern des Dosier- und Betätigungsknopfs 8 dennoch den Sperrring 45 aufnehmen zu können. Durch den für die Betätigung erforderlichen Druck des Verwenders federt der Dosier- und Betätigungsknopf 8 nämlich in der vorderen Endposition des Übertragungselements 10 und der Kolbenstange 4 in sich ein klein wenig ein, was als angenehm empfunden wird. Da der Sperrring 45 von dem Federelement 49 gegen die Außenmantelfläche des Dosier- und Betätigungsknopfs 8 gespannt wird, fährt der Sperrring 45 radial in die nun für ihn zugängliche Nut 9 ein. Die im Vergleich zum Sperrring 45 größere Breite der Nut 9 in Kombination mit der axialen Einfederung des Dosier- und Betätigungsknopfs 8 erhöht die Sicherheit, dass der Sperrring 45 auch tatsächlich in die Nut 9 einfährt und die Dosier- und Betätigungseinrichtung 7/8/10 blockiert wird. Der Sperrring 45 bildet einen Axialanschlag für die Einfederbewegung des Dosier- und Betätigungsknopfs 8. Diametral dem Federelement 49 gegenüberliegend wird durch das Einfahren des Sperrrings 45 dessen Resetgegennocken 46 radial auf die Innenmantelfläche der Dosisanzeigehülse 40 zu bewegt. Da der Sperrring 45 in dem Sperrringhalter 47 axial nicht bewegbar mit dem Mechanikhalter 5 verbunden ist, kann der Dosier- und Betätigungsknopf 8 und damit zusammen die Kolbenstange 4 nicht wieder gegen die Vorschubrichtung V zurückbewegt werden, d.h. der Sperrring 45 und der Dosier- und Betätigungsknopf 8 sind in einem Sperreingriff. Der in der Nut 9 sitzende Sperrring 45 bildet im Sperreingriff in der vordersten Position der Dosier- und Betätigungseinrichtung 7/8/10 somit eine Axialsicherung für die Dosier- und Betätigungseinrichtung 7/8/10, das geteilte Dosierglied 11/12 und die Kolbenstange 4. Die Rückstellfeder 36 ist in diesem Endzustand axial auf Druck gespannt. Eine Entspannung der Rückstellfeder 36 wird durch die von dem Sperrring 45 gebildete Axialsicherung verhindert. Durch diese Axialsicherung wird sichergestellt, dass eine erneute Produktauswahl nur aus einer definierten Position der Dosisanzeigehülse 40 vorgenommen werden kann. Diese definierte Position ist vorzugsweise, wie im Ausführungsbeispiel, die Nulldosis-Position, d.h. die Position, in der durch das Fenster 3 auf der Dosisskala 41 die Nulldosis ablesbar ist.

Um das entleerte Reservoir R gegen ein neues Reservoir R auszutauschen, werden die beiden Gehäuseabschnitte 1 und 2 auseinandergeschraubt. Aufgrund des Schraubvorgangs findet eine axiale Relativbewegung zwischen den Gehäuseabschnitten 1 und 2 statt. Durch die gespannte Reservoirhaltefeder 37 wird der Reservoirhalter 30 bei der das Gehäuse 1/2 verlängernden Bewegung der Gehäuseabschnitte 1 und 2 relativ zu dem Kolbenstangenhalter 6 in die Vorschubrichtung bis gegen einen von dem hinteren Gehäuseabschnitt 2 gebildeten Anschlag gedrückt. Zwischen einem vorderen Ende des Reservoirhalters 30 und dem vom hinteren Gehäuseabschnitt 2 gebildeten Anschlag verbleibt vor Beginn der Schraubbewegung axial ein ausreichend großer, lichter Abstand, um die Axialbewegung des Reservoirhalters 30 relativ zu dem Kolbenstangenhalter 6 zu ermöglichen.
Der Reservoirhalter 30 nimmt bei seiner relativ zu dem Kolbenstangenhalter 6 stattfindenden Axialbewegung den Mitnehmer 25 und die Gleithülse 20 mit. Aufgrund der Axialbewegung der Gleithülse 20 fahren die beiden Gleitstücke 15 und 16 an den schrägen Führungsbahnen 21 und 22 der Gleithülse 20 nach radial auswärts. Bei dieser Bewegung nach radial auswärts werden die Gleitstücke 15 und 16 von dem Mechanikhalter 5 und dem Kolbenstangenhalter 6 geradgeführt. Da das Gleitstück 15 das erste Dosierglied 11 und das Gleitstück 16 das zweite Dosierglied 12 mit ihren Stegen 17 hintergreifen, werden das erste Dosierglied 11 und das zweite Dosierglied 12 in gleicher Weise radial auseinander und dadurch aus dem Gewindeeingriff mit der Kolbenstange 4 bewegt. Die Axialbewegung, die der hintere Gehäuseabschnitt 2 relativ zu dem vorderen Gehäuseabschnitt 1 ausführt, um den Eingriff der Kolbenstange 4 und der Dosierglieder 11 und 12 zu lösen, kann als Ausrückbewegung bezeichnet werden. Durch die Ausrückbewegung werden die Dosierglieder 11 und 12 je in eine von der Kolbenstange 4 abgerückte Position bewegt. Durch die entgegengerichtete Einrückbewegung, bei der der hintere Gehäuseabschnitt 2 relativ zu dem ersten Gehäuseabschnitt 2 eine Axialbewegung in die umgekehrte Richtung ausführt, werden die Dosierglieder 11 und 12 durch das von der Gleithülse 20 und den Gleitstücken 15 und 16 gebildete Kurvengetriebe wieder in den Eingriff mit der Kolbenstange 4 bewegt. Die Ausrückbewegung und die Einrückbewegung findet aus dem komplett zusammengeschraubten Zustand der Gehäuseabschnitte 1 und 2 heraus in einem ersten Abschnitt der Schraubbewegung statt. Dieser erste Abschnitt der Schraubbewegung ist beendet, wenn der Reservoirhalter 30 von der Reservoirhaltefeder 37 gegen den vom hinteren Gehäuseabschnitt 2 gebildeten Anschlag gedrückt wird.

Fig. 3 zeigt die Vorrichtung in einem Zustand, in dem der Gewindeeingriff des geteilten Dosierglieds 11/12 mit der Kolbenstange 4 gelöst und die Kolbenstange 4 somit in dem Kolbenstangenhalter 6 axial frei verschiebbar ist. Der hintere Gehäuseabschnitt 2 hat gerade seine Ausrückbewegung vollständig ausgeführt. In dem gezeigten Zustand ist die Kolbenstange 4 bereits bis in ihre hinterste Position zurückgesetzt worden. So kann die Kolbenstange 4 beispielsweise durch leichtes Kippen der gesamten Vorrichtung von dem Kolbenstangenhalter 6 geführt in die gezeigte Endposition zurückgleiten. An dem hinteren Ende des Dosierknopfs 8 ragt in Vorschubrichtung einwärts ein Gummistopfen ab, der die Gleitbewegung der Kolbenstange 4 schonend dämpft.

Bei dem weiteren Auseinanderschrauben der Gehäuseabschnitte 1 und 2. ändern sich die von den Dosiergliedem 11 und 12, den Gleitstücken 15 und 16, der Gleithülse 20 und dem Reservoirhalter 30 relativ zu dem hinteren Gehäuseabschnitt 2 eingenommenen Positionen nicht mehr. Hierfür sorgt der Anschlag des Reservoirhalters 30 an dem hinteren Gehäuseabschnitt 2. Die beiden Gehäuseabschnitte 1 und 2 können komplett auseinandergeschraubt und das verbrauchte Reservoir R gegen ein neues ausgetauscht werden. Nach dem Einsetzen eines neuen Reservoirs R in den vorderen Gehäuseabschnitt 1 werden die beiden Gehäuseabschnitte 1 und 2 wieder zusammengeschraubt. In dem letzten Abschnitt der Schraubbewegung führt der hintere Gehäuseabschnitt 2 relativ zu dem vorderen Gehäuseabschnitt 1 seine Einrückbewegung aus, bei der der Reservoirhalter 30 in Kontakt mit dem Reservoir R oder einem Reservoirhalter gelangt und von diesem gegen die Federkraft der Reservoirhaltefeder 37 axial auf den Kolbenstangenhalter 6 zu gedrückt wird. Bei seiner Axialbewegung drückt der Reservoirhalter 30 über den Mitnehmer 25 auch die Gleithülse 20 gegen die Vorschubrichtung V nach hinten. Hierdurch werden die Gleitstücke 15 und 16 über ihren Führungseingriff mit der Gleithülse 20 nach radial einwärts bewegt bis der synchrone Eingriff der Dosierglieder 11 und 12 mit der Kolbenstange 4 wieder hergestellt ist.

Wenn im vorstehenden die Rücksetzung der Kolbenstange 4 lediglich für den Fall des restlosen Verbrauchs des Reservoirs R beschrieben wird, so ist doch anzumerken, dass die erfindungsgemäße Rücksetzung auch aus jeder anderen Axialposition der Kolbenstange 4 vorgenommen werden kann, in der die Kolbenstange 4 durch eine Dosierdrehbewegung des geteilten Dosierglieds 11/12 aus ihrer in Figur 1 dargestellten, hintersten Position bewegt worden ist.

Wenn der Dosier- und Betätigungsknopf 8 wie in den Fig. 2 und 3 bis in seine vorderste Position in den hinteren Gehäuseabschnitt 2 hineingedrückt ist, besteht zwischen der Dosierhülse 7 und dem Dosier- und Betätigungsknopf 8 keine Verdrehsicherung mehr, d.h. die Dosierhülse 7 ist relativ zu dem Dosier- und Betätigungsknopf 8 und zu dem hinteren Gehäuseabschnitt 2 frei um die Längsachse L verdrehbar. Allerdings besteht nach wie vor die Verdrehsicherung zwischen der Dosierhülse 7 und der Dosisanzeigehülse 40. Um den Dosier- und Betätigungsknopf 8 und damit zusammen die Dosier- und Betätigungseinrichtung 7/8/10 und das geteilte Dosierglied 11/12 wieder in die Dosisauswahlposition zurück zu versetzten, wird die Dosierhülse 7 um die Längsachse L in eine die Dosisanzeigehülse 40 in die Nulldosis-Position zurückführende Drehrichtung gedreht. Bei dieser Drehbewegung wird die Dosisanzeigehülse 40 relativ zu dem Mechanikhalter 5 verdreht. Infolge des Gewindeeingriffs mit dem Mechanikhalter 5 vollführt die Dosisanzeigehülse 40 eine translatorische und rotatorische Bewegung relativ zu dem Mechanikhalter 5 und dem hinteren Gehäuseabschnitt 2 in Richtung auf ihre Nulldosis-Position zu. In einem letzten Bewegungsabschnitt vor Erreichen der Nulldosis-Position, dessen Länge einer einzigen einstellbaren Dosiseinheit entspricht, kommt der Resetnocken 44 der Dosisanzeigehülse 40 in radiale Überdeckung mit dem Resetgegennocken 46 des Sperrrings 45. Der Resetnocken 44 ist an der Seite, die bei der Rückdrehbewegung der Dosisanzeigehülse 40 gegen den Resetgegennocken 46 drückt, in Umfangsrichtung verjüngt. Durch die Verjüngung wird ein allmähliches, weiches Überschieben der beiden Nocken 44 und 46 ermöglicht. Durch den im Bereich seiner Verjüngung sich allmählich nach radial einwärts verlängernden Resetnocken 44 wird der Sperrring 45 radial gegen die Rückstellkraft des Federelements 49 aus der Nut 9 herausbewegt. Die Axialsicherung ist somit gelöst, und die Rückstellfeder 36 drückt die Dosier- und Betätigungseinrichtung 7/8/10 zusammen mit der Kolbenstange 4 in die in Fig. 1 gezeigte Ausgangsposition zurück.

Die Sequenz: Auswahl der Dosis, Ausschüttung der ausgewählten Dosis durch Betätigung der Dosier- und Betätigungseinrichtung 7/8/10, Axialsicherung durch den Sperrring 45 mit Nocken 46, Rückdrehung der Dosisanzeigenhülse 40 in die Nulldosis-Position und hierdurch bewirkte Lösung der Axialsicherung und Rückfederung der Dosier- und Betätigunseinrichtung 7/8/10 in die Ausgangsposition kann wiederholt werden, bis das Reservoir R entleert ist. Durch das Zusammenwirken von Nocken 46 und Resetnocken 44 wird die Bewegung der Dosier- und Betätigunseinrichtung 7/8/10 zurück in die Ausgangsposition, aus der erneut eine Dosis ausgewählt werden kann, mit der Nulldosis-Position der Dosisanzeigehülse 40 gekoppelt. Vorteilhafterweise ist diese Kopplung so gestaltet, dass einerseits die Bewegung der Dosier- und Betätigungseinrichtung 7/8/10 nur möglich ist, wenn die Dosisanzeigenhülse 40 ihre Nulldosis-Position einnimmt, dass andererseits aber zum Auslösen der Rücksetzbewegung der Dosier- und Betätigungseinrichtung 7/8/10 keine weiteren Handgriffe erforderlich sind.

Die Produktausschüttvorrichtung des Ausführungsbeispiels ist für einen Inhalator vorgesehen, mit dem Insulin über die Atemwege verabreicht wird. Die Vorrichtung dient der Dosierung und Ausschüttung des Produkts in eine Vernebelungskammer. Das in der Vernebelungskammer somit dosiert vorliegende Produkt wird von einer Vernebelungseinrichtung vernebelt bzw. zerstäubt und durch einen Kammerauslass über die Atemwege, vorzugsweise oral, verabreicht. Das vordere Ende des vorderen Gehäuseabschnitts 1 ist mit einem als Gewinde gebildeten Anschluss G versehen, um die Vorrichtung per Gewindeeingriff an die Vernebelungskammer anschließen zu können.

Die Vorrichtung ist ohne weiteres auch unmittelbar als Injektionsgerät verwendbar, indem auf das vordere Ende des vorderen Gehäuseabschnitts 1 ein Nadelhalter mit integrierter Injektionsnadel von vorzugsweise 30 G oder dünner, beispielsweise 31 G, aufgeschraubt wird. Die Vorrichtung könnte auch für einen Druckinjektor vorgesehen sein und in diesem Fall an eine Ausstoßeinrichtung des Druckinjektors angeschlossen werden. Die Vorrichtung würde der Ausstoßeinrichtung die ausgewählte Produktdosis zuführen, und die Ausstoßeinrichtung würde diese Produktdosis unter hohem Druck durch eine Injektionsdüse ausstoßen.

### Bezugszeichen:

- 1: erster Gehäuseabschnitt
- 2: Rücksetz-Betätigungselement, zweiter Gehäuseabschnitt
- 3: Fenster
- 4: Kolbenstange
- 5: Mechanikhalter
- 6: Kolbenstangenhalter
- 7: Dosierhülse
- 8: Dosier- und Betätigungsknopf
- 8a: Betätigungsteil
- 8b: Befestigungsteil
- 8c: Endknopf
- 8d: Feder
- 9: Ausnehmung, Nut
- 10: Übertragungselement
- 10a: Querführungsbahn
- 10b: Querführungsbahn
- 11: erstes Dosierglied
- 12: zweites Dosierglied
- 13: Eingriffsbahn
- 14: ./.
- 15: Getriebeausgangsglied, Gleitstück
- 16: Getriebeausgangsglied, Gleitstück
- 17: Steg, Führungsbahn
- 18: Eingriffsbahn
- 19: ./.
- 20: Getriebeeingangsglied, Gleithülse
- 21: Führungsbahn
- 22: Führungsbahn
- 23: Zunge
- 24: Mitnehmernocken
- 25: Mitnehmer
- 26: Schulter
- 27: Zunge
- 28: Ausnehmung
- 29: ./.
- 30: Reservoirhalter
- 31: Schulter
- 32: Schuh
- 33: Seitenführung
- 34: Nocken
- 35: ./.
- 36: Rückstellfeder
- 37: Federelement, Reservoirhaltefeder
- 38: Toleranzausgleichsfeder
- 39: ./.
- 40: Dosisanzeigehülse
- 41: Dosisskala
- 42: Gewinde
- 43: Verdrehsicherung, Nocken
- 44: Resetnocken
- 45: Sperrglied, Sperrring
- 46: Resetgegennocken
- 47: Sperrringhalter
- 48: Ausnehmung
- 49: Federelement
- 50: ./.
- 51: Ausnehmung
- 52: Ausnehmung
- 53: Gewinde
- 54: Verdrehsicherungsfläche
- 55: Nocken
- 56: Anschlag
- 57-60: ./.
- 58: Schulter
- 61: Schulter
- 62: Vorsprung
- 63: Zunge
- 64: Nocken
- A: Auslass
- G: Anschluss
- H₁: Abstand Kolben/Kolbenstange
- H₂: Hub
- K: Kolben
- L: Längsachse
- R: Reservoir
- V: Vorschubrichtung, Betätigungsrichtung

## Patentansprüche

1. Produktausschüttvorrichtung mit Kolbenstangen-Eilrücksetzung, das Gerät umfassend:
a) ein Gehäuse (1/2) mit einem ersten Gehäuseabschnitt (1) für ein mit Produkt gefülltes Reservoir (R), aus dem das Produkt mittels eines Kolbens (K) ausschüttbar ist,
b) eine Kolbenstange (4), die in eine Vorschubrichtung (V) bewegbar ist, um den Kolben (K) für die Ausschüttung des Produkts in Richtung auf einen Auslass (A) des Reservoirs (R) zu bewegen,
c) eine Dosier- und Betätigungseinrichtung (7/8/10), die relativ zu dem Gehäuse (1/2) eine Dosierbewegung und gemeinsam mit der Kolbenstange (4) relativ zu dem Gehäuse (1/2) eine Ausschüttbewegung in die Vorschubrichtung (V) ausführt,
d) ein Dosierglied (11), das mit der Dosier- und Betätigungseinrichtung (7/8/10) so verbunden ist, dass es die Ausschüttbewegung mitmacht und bei der Dosierbewegung auch eine Dosierbewegung ausführt,
e) wobei das Dosierglied (11) quer zu der Kolbenstange (4) bewegbar ist, um den Eingriff mit der Kolbenstange (4) für eine Rücksetzung der Kolbenstange (4) entgegen der Vorschubrichtung (V) zu lösen,
f) und ein Rücksetz-Betätigungselement (2), das mit dem Gehäuse (1/2), vorzugsweise mit dem ersten Gehäuseabschnitt (1) bewegbar verbunden ist, um eine Ausrückbewegung und eine Einrückbewegung auszuführen,
**dadurch gekennzeichnet, dass**
g) das Dosierglied (11) mit der Kolbenstange (4) in einem formschlüssigen Eingriff ist, der die Dosierbewegung des Dosierglieds (11) relativ zu der Kolbenstange (4) zulässt, und die Mitnahme der Kolbenstange (4) bei der Ausschüttbewegung bewirkt,
h) das Dosierglied (11) ein mit der Kolbenstange (4) in einem Gewindeeingriff befindliches Gewindeglied ist oder die Kolbenstange stattdessen als Zahnstange und das Dosierglied als elastischer Schnapper ausgebildet ist,
i) ein Kurvengetriebe (10/15/20) vorgesehen ist, das das Rücksetz-Betätigungselement (2) mit dem Dosierglied (11) so koppelt, dass das Dosierglied (11) durch die Ausrückbewegung aus dem Eingriff und durch die Eindrückbewegung in den Eingriff mit der Kolbenstange (4) bewegt wird,
j) das Kurvengetriebe (10/15/20) eine quer zu der Längsachse (L) weisende Querführungsbahn (10a, 10b) bildet, an der das Dosierglied (11) bei der Bewegung in und aus dem Eingriff geführt wird.

2. Produktausschüttvorrichtung nach Anspruch 1, **dadurch gekennzeichnet, dass** ein zweiter Gehäuseabschnitt (2), der mit ersten Gehäuseabschnitt (1) bewegbar verbunden ist, das Rücksetz-Betätigungselement (2) bildet.

3. Produktausschüttvorrichtung nach einem der vorhergehenden Ansprüche, **dadurch gekennzeichnet, dass** die Ausrückbewegung und die Einrückbewegung je eine Relativbewegung sind, die zwischen dem Rücksetz-Betätigungselement (2) und dem ersten Gehäuseabschnitt (1) entlang einer Längsachse (L) des ersten Gehäuseabschnitts (1) stattfindet.

4. Produktausschüttvorrichtung nach einem der vorhergehenden Ansprüche, **dadurch gekennzeichnet, dass** der erste Gehäuseabschnitt (1) und das Rücksetz-Betätigungselement (2) in einem Gewindeeingriff um eine Längsachse (L) des ersten Gehäuseabschnitts (1) sind und die Ausrückbewegung und die Einrückbewegung je eine durch den Gewindeeingriff bestimmt Schraubbewegung ist.

5. Produktausschüttvorrichtung nach einem der vorhergehenden Ansprüche, **dadurch gekennzeichnet, dass** das Getriebe (10/5/20) ein mit dem Rücksetz-Betätigungselement (2) verbundenes Getriebeeingangsglied (20) und ein mit dem Dosierglied (11) verbundenes Getriebeausgangsglied (15) umfasst, wobei das Getriebeeingangsglied (20) und das Getriebeausgangsglied (15) in einem Eingriff sind, der eine durch die Einrückbewegung und die Ausrückbewegung des Rücksetz-Betätigungselements (2) bewirkte Bewegung, die das Getriebeeingangsglied (20) relativ zu dem Getriebeausgangsglied (15) ausführt, in eine Bewegung des Getriebeausgangsglieds (15) quer zu der Kolbenstange (4) umwandelt.

6. Produktausschüttvorrichtung nach dem vorhergehenden Anspruch, **dadurch gekennzeichnet, dass** von dem Getriebeeingangsglied (15) und dem Getriebeausgangsglied (20) das eine (20) eine weitere Führungsbahn (21) und das andere (15) in dem Eingriff ein an der weiteren Führungsbahn (21) geführtes Eingriffsglied bildet.

7. Produktausschüttvorrichtung nach dem vorhergehenden Anspruch, **dadurch gekennzeichnet, dass** die weitere Führungsbahn (21) zu der Kolbenstange (4) in Längsrichtung (L) der Kolbenstange (4) eine Neigung aufweist.

8. Produktausschüttvorrichtung nach einem der zwei vorhergehenden Ansprüche, **dadurch gekennzeichnet, dass** die weitere Führungsbahn (21) so verläuft, dass eine zwischen dem Getriebeeingangsglied (20) und dem Getriebeausgangsglied (15) entlang einer Längsachse (L) der Kolbenstange (4) stattfindende Relativbewegung durch den Führungseingriff die Relativbewegung quer zu der Kolbenstange (4) bewirkt.

9. Produktausschüttvorrichtung nach einem der drei vorhergehenden Ansprüche, **dadurch gekennzeichnet, dass** das Getriebeeingangsglied (20) und das Getriebeausgangsglied (15) an einander zugewandten Seiten konisch sind.

10. Produktausschüttvorrichtung nach einem der Ansprüche 5-9, **dadurch gekennzeichnet, dass** das Getriebeausgangsglied (15) das Dosierglied (11) bei der Bewegung quer zu der Kolbenstange (4) mitnimmt.

11. Produktausschüttvorrichtung nach dem vorhergehenden Anspruch, **dadurch gekennzeichnet, dass** das Getriebeausgangsglied (15) das Dosierglied (11) hintergreift, um es bei der Bewegung quer zu der Kolbenstange (4) mitzunehmen.

12. Produktausschüttvorrichtung nach einem der Ansprüche 5-11, **dadurch gekennzeichnet, dass** das Dosierglied (11) in und gegen die Vorschubrichtung (V) relativ zu dem Getriebeausgangsglied (15) bewegbar ist.

13. Produktausschüttvorrichtung nach einem der Ansprüche 5-12, **dadurch gekennzeichnet, dass** das Getriebeeingangsglied (20) mit einem Reservoirhalter (30), der in Vorschubrichtung (V) gegen ein das Reservoir (R) bildendes Behältnis gedrückt wird, verbunden ist, wobei die Verbindung die Mitnahme des Getriebeeingangsglieds (20) bewirkt, wenn sich der Reservoirhalter (30) relativ zu dem Dosierglied (11) entlang einer Längsachse (L) der Kolbenstange (4) bewegt, aber eine Drehbewegung des Getriebeeingangsglieds (20) um die Längsachse (L) der Kolbenstange (4) zulässt.

14. Produktausschüttvorrichtung nach einem der Ansprüche 5-13, **dadurch gekennzeichnet, dass** das Dosierglied (11), das Getriebeausgangsglied (15) und das Getriebeeingangsglied (20) um die Längsachse (L) der Kolbenstange (4) verdrehgesichert miteinander verbunden sind.

15. Produktausschüttvorrichtung nach einem der Ansprüche 5-14, **dadurch gekennzeichnet, dass** ein Federelement (37) vorgesehen ist, das durch die Einrückbewegung des Rücksetz-Betätigungselement (2) gespannt wird und bei der Ausrückbewegung aufgrund seiner Spannkraft die Bewegung des Getriebeeingangsglieds (20) relativ zu dem Getriebeausgangsglied (15) bewirkt.

16. Produktausschüttvorrichtung nach dem vorhergehenden Anspruch, **dadurch gekennzeichnet**, das ein von dem ersten Gehäuseabschnitt (1) aufgenommenes Behältnis das Reservoir (R) bildet und das Federelement (37) das Behältnis in die Vorschubrichtung (V) gegen einen von dem ersten Gehäuseabschnitt (1) gebildeten Anschlag drückt.

17. Produktausschüttvorrichtung nach einem der vorhergehenden Ansprüche, **dadurch gekennzeichnet**, das die Dosier- und Betätigungseinrichtung (7/8/10) ein die Kolbenstange (4) umgebendes, hülsenförmiges Übertragungselement (10) umfasst und das Übertragungselement (10) das Dosierglied (15) axial verschiebegesichert und quer zu der Kolbenstange (4) bewegbar lagert.

18. Produktausschüttvorrichtung nach dem vorhergehenden Anspruch, **dadurch gekennzeichnet, dass** das Übertragungselement (10) die Querführungsbahn (10a, 10b) bildet.

19. Produktausschüttvorrichtung nach einem der vorhergehenden Ansprüche, **dadurch gekennzeichnet, dass** die Dosier- und Betätigungseinrichtung (7/8/10) ihre Dosierbewegung relativ zu der Kolbenstange (4) ausführt und mit dem Dosierglied (11) so verbunden ist, dass das Dosierglied (11) die Dosierbewegung der Dosier- und Betätigungseinrichtung (7/8/10) mitmacht.

20. Produktausschüttvorrichtung nach einem der vorhergehenden Ansprüche, **dadurch gekennzeichnet, dass** das Dosierglied (11) und ein weiteres Dosierglied (12) ein axial geteilte Gewindemutter (11/12) bilden, vorzugsweise eine axial geteilte Gewindemutter (11/12), die mit der als Gewindestange gebildeten Kolbenstange (4) in einem Gewindeeingriff ist, und dass die Anordnung der Dosierglieder (11, 12) an einander gegenüberliegenden Längsseiten der Kolbenstange (4) symmetrisch ist.

21. Produktausschüttvorrichtung nach einem der vorhergehenden Ansprüche, **dadurch gekennzeichnet, dass** die Vorrichtung für eine Verwendung als Dosiereinrichtung für einen Inhalator vorgesehen ist, um einer Vernebelungseinrichtung des Inhalators eine ausgewählte Produktdosis zuzuführen.

22. Injektionsgerät mit einer Produktausschüttvorrichtung nach einem der vorhergehenden Ansprüche und einer mit dem Auslass (A) des Reservoirs (R) verbundenen Einstechnadel oder Druckinjektionseinrichtung.

## Claims

1. A product delivery device with rapid piston rod resetting, said device comprising:
a) a casing (1/2) comprising a first casing portion (1) for a reservoir (R) which is filled with product and from which the product can be delivered by means of a piston (K);
b) a piston rod (4) which can be moved in an advancing direction (V) in order to move the piston (K) towards an outlet (A) of the reservoir (R) in order to deliver the product;
c) a dosing and activating means (7/8/10) which performs a dosing movement relative to the casing (1/2) and, together with the piston rod (4), performs a delivery movement in the advancing direction (V), relative to the casing (1/2);
d) a dosing member (11) which is connected to the dosing and activating means (7/8/10) such that it is slaved in the delivery movement and, during the dosing movement, also performs a dosing movement;
e) wherein the dosing member (11) can be moved transversely with respect to the piston rod (4), in order to release the engagement with the piston rod (4) for resetting the piston rod (4) counter to the advancing direction (V);
f) and a resetting and activating element (2) which is movably connected to the casing (1/2), preferably to the first casing portion (1), in order to perform a disengaging movement and an engaging movement;
**characterised in that**
g) the dosing member (11) is in a positive-lock engagement with the piston rod (4) which permits the dosing movement of the dosing member (11) relative to the piston rod (4), and which slaves the piston rod (4) during the delivery movement;
h) the dosing member (11) is a threaded member in threaded engagement with the piston rod (4), or the piston rod is instead formed as a toothed rod and the dosing member is formed as an elastic catch;
i) a cam gear (10/15/20) is provided which couples the resetting and activating element (2) to the dosing member (11), such that the dosing member (11) is moved out of engagement with the piston rod (4) by the disengaging movement and into engagement with the piston rod (4) by the engaging movement;
j) the cam gear (10/15/20) forms a transverse guiding rail (10a, 10b) which points transversely with respect to the longitudinal axis (L) and on which the dosing member (11) is guided when moving into and out of engagement.

2. The product delivery device according to claim 1, **characterised in that** a second casing portion (2), which is movably connected to the first casing portion (1), forms the resetting and activating element (2).

3. The product delivery device according to any one of the preceding claims, **characterised in that** the disengaging movement and the engaging movement are each relative movements between the resetting and activating element (2) and the first casing portion (1) along a longitudinal axis (L) of the first casing portion (1).

4. The product delivery device according to any one of the preceding claims, **characterised in that** the first casing portion (1) and the resetting and activating element (2) are in threaded engagement about a longitudinal axis (L) of the first casing portion (1), and the disengaging movement and the engaging movement are each a screwing movement defined by the threaded engagement.

5. The product delivery device according to any one of the preceding claims, **characterised in that** the gear (10/15/20) comprises a gear input member (20) connected to the resetting and activating element (2) and a gear output member (15) connected to the dosing member (11), wherein the gear input member (20) and the gear output member (15) are in an engagement which converts a movement caused by the engaging movement and the disengaging movement of the resetting and activating element (2), which the gear input member (20) performs relative to the gear output member (15), into a movement of the gear output member (15) transversely with respect to the piston rod (4).

6. The product delivery device according to the preceding claim, **characterised in that** one (20) of the gear input member (20) and the gear output member (15) forms an additional guiding rail (21) and, in engagement, the other (15) of the gear input member (20) and the gear output member (15) forms an engagement member guided on the additional guiding rail (21).

7. The product delivery device according to the preceding claim, **characterised in that** the additional guiding rail (21) exhibits an inclination with respect to the piston rod (4) in the longitudinal direction (L) of the piston rod (4).

8. The product delivery device according to any one of the preceding two claims, **characterised in that** the additional guiding rail (21) runs such that a relative movement between the gear input member (20) and the gear output member (15) along a longitudinal axis (L) of the piston rod (4) causes the relative movement transversely with respect to the piston rod (4), by means of the guiding engagement.

9. The product delivery device according to any one of the preceding three claims, **characterised in that** the gear input member (20) and the gear output member (15) are conical on mutually facing sides.

10. The product delivery device according to any one of claims 5 to 9, **characterised in that** the gear output member (15) slaves the dosing member (11) when it moves transversely with respect to the piston rod (4).

11. The product delivery device according to the preceding claim, **characterised in that** the gear output member (15) grips behind the dosing member (11), in order to slave it when it moves transversely with respect to the piston rod (4).

12. The product delivery device according to any one of claims 5 to 11, **characterised in that** the dosing member (11) can be moved in and counter to the advancing direction (V), relative to the gear output member (15).

13. The product delivery device according to any one of claims 5 to 12, **characterised in that** the gear input member (20) is connected to a reservoir holder (30) which is pressed in the advancing direction (V) against a container forming the reservoir (R), wherein said connection slaves the gear input member (20) when the reservoir holder (30) is moved relative to the dosing member (11) along a longitudinal axis (L) of the piston rod (4), but permits the gear input member (20) to rotate about the longitudinal axis (L) of the piston rod (4).

14. The product delivery device according to any one of claims 5 to 13, **characterised in that** the dosing member (11), the gear output member (15) and the gear input member (20) are connected to each other, secured against rotating about the longitudinal axis (L) of the piston rod (4).

15. The product delivery device according to any one of claims 5 to 14, **characterised in that** a spring element (37) is provided which is tensed by the engaging movement of the resetting and activating element (2) and, during the disengaging movement, moves the gear input member (20) relative to the gear output member (15), due to its tension force.

16. The product delivery device according to the preceding claim, **characterised in that** a container accommodated by the first casing portion (1) forms the reservoir (R), and the spring element (37) presses the container in the advancing direction (V) against a stopper formed by the first casing portion (1).

17. The product delivery device according to any one of the preceding claims, **characterised in that** the dosing and activating means (7/8/10) comprises a sleeve-shaped transfer element (10) which surrounds the piston rod (4), and the transfer element (10) mounts the dosing member (11) such that it is secured against axially shifting and can be moved transversely with respect to the piston rod (4).

18. The product delivery device according to the preceding claim, **characterised in that** the transfer element (10) forms the transverse guiding rail (10a, 10b).

19. The product delivery device according to any one of the preceding claims, **characterised in that** the dosing and activating means (7/8/10) performs its dosing movement relative to the piston rod (4) and is connected to the dosing member (11) such that the dosing member (11) is slaved in the dosing movement of the dosing and activating means (7/8/10).

20. The product delivery device according to any one of the preceding claims, **characterised in that** the dosing member (11) and another dosing member (12) form an axially split threaded nut (11/12) which is in threaded engagement with the piston rod (4) which is formed as a threaded rod, and **in that** the dosing members (11, 12) are arranged symmetrically on mutually opposing longitudinal sides of the piston rod (4).

21. The product delivery device according to any one of the preceding claims, **characterised in that** the device is provided for use as a dosing means for an inhaler, in order to supply a selected product dosage to an atomising means of the inhaler.

22. An injection apparatus comprising a product delivery device according to any one of the preceding claims, and an injection needle or pressure injection means connected to the outlet (A) of the reservoir (R).

## Revendications

1. Dispositif de déversement de produit comportant un mécanisme de retour rapide de la tige de piston, l'appareil comprenant :
a) un boîtier (1/2) comportant une première partie de boîtier (1) pour un réservoir (R) rempli de produit, réservoir hors duquel le produit peut être déversé au moyen d'un piston (K),
b) une tige de piston (4) qui peut se déplacer dans une direction d'avance (V) afin de déplacer le piston (K) en direction d'un orifice de sortie (A) du réservoir (R) en vue de déverser le produit,
c) un système d'actionnement et de dosage (7/8/10) qui effectue un mouvement de dosage par rapport au boîtier (1/2) et, conjointement avec la tige de piston (4), un mouvement de déversement dans la direction d'avance (V) par rapport au boîtier (1/2),
d) un organe de dosage (11) qui est relié de telle manière au système d'actionnement et de dosage (7/8/10) qu'il effectue également le mouvement de déversement et également un mouvement de dosage lors du mouvement de dosage,
e) l'organe de dosage (11) pouvant se déplacer transversalement par rapport à la tige de piston (4) afin de libérer la prise avec la tige de piston (4) pour un retour de la tige de piston (4) en sens inverse à la direction d'avance (V),
f) et un élément d'actionnement de retour (2) qui est relié de manière mobile au boîtier (1/2), de préférence à la première partie de boîtier (1), afin d'effectuer un mouvement de déclenchement et un mouvement d'enclenchement,
**caractérisé en ce que**
g) l'organe de dosage (11) est en prise par correspondance de forme avec la tige de piston (4), laquelle prise permet le mouvement de dosage de l'organe de dosage (11) par rapport à la tige de piston (4), et provoque l'entraînement de la tige de piston (4) lors du mouvement de déversement,
h) l'organe de dosage (11) est un organe fileté qui est en prise par filetage avec la tige de piston (4) ou, au lieu de cela, la tige de piston est conçue comme crémaillère et l'organe de dosage comme élément d'enclenchement élastique,
i) il est prévu un mécanisme à came (10/15/20), qui couple l'élément d'actionnement de retour (2) à l'organe de dosage (11) de manière à ce que l'organe de dosage (11) soit déplacé hors de la prise avec la tige de piston (4) par le mouvement de déclenchement, et en prise avec la tige de piston (4) par le mouvement d'enclenchement,
j) le mécanisme à came (10/15/20) forme une voie de guidage transversal (10a, 10b) dirigée transversalement par rapport à l'axe longitudinal (L), sur laquelle l'organe de dosage (11) est guidé par le mouvement en prise et hors de la prise.

2. Dispositif de déversement de produit selon la revendication 1, **caractérisé en ce qu'**une deuxième partie de boîtier (2), laquelle est reliée de manière mobile à la première partie de boîtier (1), forme l'élément d'actionnement de retour (2).

3. Dispositif de déversement de produit selon l'une quelconque des revendications précédentes, **caractérisé en ce que** le mouvement de déclenchement et le mouvement d'enclenchement sont chacun un mouvement relatif qui se produit entre l'élément d'actionnement de retour (2) et la première partie de boîtier (1) le long d'un axe longitudinal (L) de la première partie de boîtier (1).

4. Dispositif de déversement de produit selon l'une quelconque des revendications précédentes, **caractérisé en ce que** la première partie de boîtier (1) et l'élément d'actionnement de retour (2) sont en prise par filetage autour d'un axe longitudinal (L) de la première partie de boîtier (1), et le mouvement de déclenchement et le mouvement d'enclenchement étant chacun un mouvement de vissage défini par la prise par filetage.

5. Dispositif de déversement de produit selon l'une quelconque des revendications précédentes, **caractérisé en ce que** le mécanisme (10/5/20) comprend un organe d'entrée de mécanisme (20) relié à l'élément d'actionnement de retour (2), et un organe de sortie de mécanisme (15) relié à l'organe de dosage (11), l'organe d'entrée de mécanisme (20) et l'organe de sortie de mécanisme (15) étant en prise, laquelle prise convertit un mouvement provoqué par le mouvement d'enclenchement et par le mouvement de déclenchement de l'élément d'actionnement de retour (2), déplaçant l'organe d'entrée de mécanisme (20) par rapport à l'organe de sortie de mécanisme (15), en un mouvement de l'organe de sortie de mécanisme (15) transversalement par rapport à la tige de piston (4).

6. Dispositif de déversement de produit selon la revendication précédente, **caractérisé en ce que** parmi l'organe d'entrée de mécanisme (15) et l'organe de sortie de mécanisme (20), l'un (20) forme une voie de guidage supplémentaire (21) et l'autre (15) un organe d'engagement guidé sur la voie de guidage supplémentaire (21) dans la prise.

7. Dispositif de déversement de produit selon la revendication précédente, **caractérisé en ce que** la voie de guidage supplémentaire (21) présente une inclinaison par rapport à la tige de piston (4) dans le sens longitudinal (L) de la tige de piston (4).

8. Dispositif de déversement de produit selon l'une quelconque des deux revendications précédentes, **caractérisé en ce que** la voie de guidage supplémentaire (21) s'étend de telle manière qu'un mouvement relatif se produisant entre l'organe d'entrée de mécanisme (20) et l'organe de sortie de mécanisme (15) le long d'un axe longitudinal (L) de la tige de piston (4) provoque, du fait de la prise de guidage, le mouvement relatif transversalement par rapport à la tige de piston (4).

9. Dispositif de déversement de produit selon l'une quelconque des trois revendications précédentes, **caractérisé en ce que** l'organe d'entrée de mécanisme (20) et l'organe de sortie de mécanisme (15) sont coniques au niveau des côtés tournés l'un vers l'autre.

10. Dispositif de déversement de produit selon l'une quelconque des revendications 5 à 9, **caractérisé en ce que** l'organe de sortie de mécanisme (15) entraîne l'organe de dosage (11) lors du mouvement transversalement par rapport à la tige de piston (4).

11. Dispositif de déversement de produit selon la revendication précédente, **caractérisé en ce que** l'organe de sortie de mécanisme (15) vient en prise derrière l'organe de dosage (11) afin de l'entraîner lors du mouvement transversalement par rapport à la tige de piston (4).

12. Dispositif de déversement de produit selon l'une quelconque des revendications 5 à 11, **caractérisé en ce que** l'organe de dosage (11) peut se déplacer dans la direction d'avance (V) et en sens inverse de celle-ci par rapport à l'organe de sortie de mécanisme (15).

13. Dispositif de déversement de produit selon l'une quelconque des revendications 5 à 12, **caractérisé en ce que** l'organe d'entrée de mécanisme (20) est relié à un support de réservoir (30), qui est pressé dans la direction d'avance (V) contre un récipient formant le réservoir (R), la liaison provoquant l'entraînement de l'organe d'entrée de mécanisme (20) lorsque le support de réservoir (30) se déplace par rapport à l'organe de dosage (11) le long d'un axe longitudinal (L) de la tige de piston (4), mais permet un mouvement rotatif de l'organe d'entrée de mécanisme (20) autour de l'axe longitudinal (L) de la tige de piston (4).

14. Dispositif de déversement de produit selon l'une quelconque des revendications 5 à 13, **caractérisé en ce que** l'organe de dosage (11), l'organe de sortie de mécanisme (15) et l'organe d'entrée de mécanisme (20) sont reliés les uns aux autres de manière fixe en rotation autour de l'axe longitudinal (L) de la tige de piston (4).

15. Dispositif de déversement de produit selon l'une quelconque des revendications 5 à 14, **caractérisé en ce qu'**il est prévu un élément formant ressort (37) qui est tendu à la suite du mouvement d'enclenchement de l'élément d'actionnement de retour (2), et qui, lors du mouvement de déclenchement, entraîne en raison de sa force élastique le mouvement de l'organe d'entrée de mécanisme (20) par rapport à l'organe de sortie de mécanisme (15).

16. Dispositif de déversement de produit selon la revendication précédente, **caractérisé en ce qu'**un récipient logé dans la première partie de boîtier (1) forme le réservoir (R), et l'élément formant ressort (37) presse le récipient dans la direction d'avance (V) contre une butée formée par la première partie de boîtier (1).

17. Dispositif de déversement de produit selon l'une quelconque des revendications précédentes, **caractérisé en ce que** le système d'actionnement et de dosage (7/8/10) comprend un élément de transmission (10) en forme de douille, entourant la tige de piston (4) et l'élément de transmission (10) loge l'organe de dosage (15) de manière fixe en translation axiale et déplaçable transversalement par rapport à la tige de piston (4).

18. Dispositif de déversement de produit selon la revendication précédente, **caractérisé en ce que** l'élément de transmission (10) forme la voie de guidage transversal (10a, 10b).

19. Dispositif de déversement de produit selon l'une quelconque des revendications précédentes, **caractérisé en ce que** le système d'actionnement et de dosage (7/8/10) effectue son mouvement de dosage par rapport à la tige de piston (4) et est relié à l'organe de dosage (11) de manière à ce que l'organe de dosage (11) effectue également le mouvement de dosage du système d'actionnement et de dosage (7/8/10).

20. Dispositif de déversement de produit selon l'une quelconque des revendications précédentes, **caractérisé en ce que** l'organe de dosage (11) et un organe de dosage supplémentaire (12) forment un écrou fileté (11/12) divisé axialement, de préférence un écrou fileté (11/12) divisé axialement, qui est en prise par filetage avec la tige de piston (4) formée comme tige filetée, et **en ce que** les organes de dosage (11, 12) sont disposés symétriquement sur les grands côtés opposés de la tige de piston (4).

21. Dispositif de déversement de produit selon l'une quelconque des revendications précédentes, **caractérisé en ce que** le dispositif est prévu pour être utilisé comme système de dosage d'un inhalateur, afin d'amener une dose de produit choisie à un système de pulvérisation de l'inhalateur.

22. Appareil d'injection comportant un dispositif de déversement de produit selon l'une quelconque des revendications précédentes et une aiguille à piquer ou un système d'injection par pression reliés à l'orifice de sortie (A) du réservoir (R).
